# EUROPEAN PATENT APPLICATION

(11) **EP 4 821 664 A1**
(43) Date of publication of application: **14.10.2026**
(21) Application number: 26157813.2
(22) Date of filing: 11.02.2026
(51) Int. Cl.: B29C 70/38, B29C 70/54, G01N 33/00

(54) **METHODS AND AUTOMATED SYSTEMS FOR LAYUP AND ANALYSIS OF COMPOSITE MATERIALS DURING FABRICATION OF COMPOSITE WORKPIECES**

(30) Priority: 09.04.2025 US 202519174210
(71) Applicant: The Boeing Company, Arlington, VA 22202 (US)
(72) Inventor: BAUER, Andrew L., Arlington, 22202 (US); REYNOLDS, Carla E., Arlington, 22202 (US); RABIEGA, Marcin A., Arlington, 22202 (US); LOUIE, Michael K., Arlington, 22202 (US); WINFREE, Troy, Arlington, 22202 (US)
(74) Representative: Boult Wade Tennant LLP

(57) **Abstract**

A method for layup and analysis of composite material during fabrication of a composite workpiece includes laying up a layer of the composite material for the composite workpiece based on design data for the layer and detecting as-built data for the layer using a quality detection device. Next, processing the as-built data for the layer to determine as-built fiber volume fill characteristics of the layer. Then, the method includes comparing the as-built fiber volume fill characteristics to as-planned fiber volume fill characteristics based on the design data for the layer to determine projected fiber volume fill ratios for the layer. An automated system for layup and analysis of composite material during fabrication of a composite workpiece includes at least one analytical computing device, a robotic manipulator, a layup mandrel and a quality detection device.

## Description

### FIELD

The present disclosure relates generally to layup and analysis of composite materials during fabrication of composite workpieces and, particularly, to methods and automated systems associated therewith. The methods include laying up multiple layers of composite material and detecting as-built data for each layer. For example, the as-built data is processed to determine as-built fiber volume fill characteristics. The as-built fiber volume fill characteristics are compared to as-planned fiber volume fill characteristics based on design data for the composite workpiece. The comparison determines projected fiber volume file ratios for each layer. The projected fiber volume file ratios for each layer can be processed to determine through thickness projected fiber volume file ratios for any combination of multiple layers.

### BACKGROUND

In general, a composite structure is a structure that is formed from two or more constituent component materials with different physical and/or chemical properties that, when combined, produce a composite material having characteristics that are different than the characteristics of the individual components materials.

As an example, one type of composite material is carbon fiber reinforced plastic ("CFRP"). CFRP may include one or more composite layers or plies laminated together to form a sheet, laminate or layup. Each of the composite layers or plies can include a reinforcement material and a matrix material. The matrix material surrounds, binds and supports the reinforcement material. The reinforcement material provides structural strength to the matrix material and the CFRP. The matrix material is generally a non-conductive polymer such as an epoxy resin. The reinforcement material generally consists of strands of carbon fiber, which are electrically conductive.

In the current state, analysis of fiber volume fill in composite workpieces is done manually with large quantities of engineering manufacturing and labor hours required to evaluate requirements. Due to the significant reliance on tedious analysis, errors may be prone to occur. Typically, this requires time consuming manual analysis as well as part build processes to evaluate the requirement. To meet a set of similar requirements has been known in some cases for large complex parts to take more than a year of successive iterations to design an NC program which meets the requirements.

Accordingly, those skilled in the art continue with research and development efforts to improve techniques for layup and analysis of composite materials during fabrication of composite workpieces.

### SUMMARY

Disclosed are examples of methods for layup and analysis of composite material during fabrication of a composite workpiece and automated systems for layup and analysis of composite material during fabrication of a composite workpiece. The following is a non-exhaustive list of examples, which may or may not be claimed, of the subject matter according to the present disclosure.

In some examples, the disclosed method for layup and analysis of composite material during fabrication of a composite workpiece includes: (1) laying up a layer of the composite material for the composite workpiece based on design data for the layer; (2) detecting as-built data for the layer using a quality detection device; (3) processing the as-built data for the layer to determine as-built fiber volume fill characteristics of the layer; and (4) comparing the as-built fiber volume fill characteristics to as-planned fiber volume fill characteristics based on the design data for the layer to determine projected fiber volume fill ratios for the layer.

In some examples, the disclosed automated system for layup and analysis of composite material during fabrication of a composite workpiece including at least one analytical computing device, a robotic manipulator, a layup mandrel and a quality detection device. The at least one analytical computing device storing a design data analysis application program, an as-built data analysis application program and a projected fiber volume fill ratio application program. The robotic manipulator in a fabrication cell and in operative communication with the at least one analytical computing device. The robotic manipulator includes a composite material placement device. The layup mandrel in the fabrication cell and configured for layup of the composite material. The quality detection device in the fabrication cell and in operative communication with the at least one analytical computing device.

In some examples, the disclosed method for layup and analysis of composite material during fabrication of a composite workpiece includes: (1) laying up a layer of multiple layers of the composite material for the composite workpiece based on design data for the layer; (2) detecting as-built data for the layer using a quality detection device; (3) processing the as-built data for the layer to determine as-built fiber volume fill characteristics of the layer; (4) comparing the as-built fiber volume fill characteristics for the layer to as-planned fiber volume fill characteristics based on the design data for the layer to determine projected fiber volume fill ratios for the layer; (5) repeating the laying up, the detecting, the processing, and the comparing for each subsequent layer of the multiple layers; and (6) processing the projected fiber volume fill ratios for the multiple layers to determine at least one through thickness projected fiber volume fill ratio.

In some examples, the disclosed method for layup and analysis of composite material during fabrication of a composite workpiece includes: (1) laying up a layer on a layup mandrel based on design data; (2) detecting as-built data using a quality detection device; (3) processing the as-built data to determine as-built fiber volume fill characteristics; and (4) comparing the as-built fiber volume fill characteristics to as-planned fiber volume fill characteristics to determine projected fiber volume fill ratios for the layer.

In some examples, the disclosed automated system for layup and analysis of composite material during fabrication of a composite workpiece including an analytical computing device, a composite material placement device, a layup mandrel and a quality detection device. The analytical computing device storing a design data analysis application program, an as-built data analysis application program and a projected fiber volume fill ratio application program. The composite material placement device in operative communication with the analytical computing device. The layup mandrel configured for layup of the composite material. The quality detection device in operative communication with the analytical computing device.

In some examples, the disclosed method for optimizing design data during fabrication of a composite workpiece includes: (1) laying up a layer on a layup mandrel based on the design data; (2) detecting as-built data using a quality detection device; (3) processing the as-built data to determine as-built fiber volume fill characteristics; (4) comparing the as-built fiber volume fill characteristics to as-planned fiber volume fill characteristics to determine projected fiber volume fill ratios) for the layer; (5) optimizing the design data to form optimized design data for at least one subsequent layer to reduce a risk probability of not achieving fiber volume fill ratio requirements based at least in part on the projected fiber volume fill ratios; and (6) laying up a second layer over the layer on the layup mandrel based on the optimized design data.

Other examples of the disclosed methods for layup and analysis of composite material during fabrication of a composite workpiece and automated systems for layup and analysis of composite material during fabrication of a composite workpiece will become apparent from the following detailed description, the accompanying drawings and the appended claims.

### BRIEF DESCRIPTION OF THE DRAWINGS

Fig. 1 is a functional block diagram of an example of an automated system for layup and analysis of composite material during fabrication of a composite workpiece;
Fig. 2 is a side view of an example of a composite workpiece;
Figs. 3A-B is a flow diagram of examples of methods for layup and analysis of composite material during fabrication of a composite workpiece;
Fig. 4, in combination with Fig. 3A, is a flow diagram of another example of a method for layup and analysis of composite material during fabrication of a composite workpiece;
Fig. 5, in combination with Figs. 3A and 4, is a flow diagram of yet another example of a method for layup and analysis of composite material during fabrication of a composite workpiece;
Fig. 6, in combination with Figs. 3A, 4 and 5, is a flow diagram of still another example of a method for layup and analysis of composite material during fabrication of a composite workpiece;
Fig. 7 is a flow diagram of an example of the optimizing of the design data of Fig. 6;
Fig. 8, in combination with Figs. 3A, 4 and 5, is a flow diagram of still yet another example of a method for layup and analysis of composite material during fabrication of a composite workpiece;
Fig. 9 is a functional block diagram of another example of another automated system for layup and analysis of composite material during fabrication of a composite workpiece;
Fig. 10 is a block diagram showing examples of design data used in conjunction with layup and analysis of composite material during fabrication of a composite workpiece;
Figs. 11A-11C are functional diagrams showing examples of plies, tows and courses of tows used in conjunction with layup of composite material during fabrication of a composite workpiece;
Figs. 12A and 12B are functional diagrams showing examples of gaps and overlaps between adjacent tows in conjunction with layup and analysis of composite material during fabrication of a composite workpiece;
Fig. 13 is a block diagram showing an example of the composite material placement device of Fig. 9;
Fig. 14 is a block diagram showing examples of as-built data used in conjunction with layup and analysis of composite material during fabrication of a composite workpiece;
Fig. 15 is a block diagram showing examples of the quality detection device of Fig. 9;
Fig. 16 is a functional diagram showing an example of a grid structure for a workpiece used in conjunction with layup and analysis of composite material during fabrication of a composite workpiece;
Fig. 17 is a flow diagram of another example of a method for layup and analysis of composite material during fabrication of a composite workpiece;
Fig. 18, in combination with Fig. 17, is a flow diagram of yet another example of a method for layup and analysis of composite material during fabrication of a composite workpiece;
Fig. 19, in combination with Figs. 17 and 18, is a flow diagram of still another example of a method for layup and analysis of composite material during fabrication of a composite workpiece;
Fig. 20, in combination with Figs. 17, 18 and 19, is a flow diagram of still yet another example of a method for layup and analysis of composite material during fabrication of a composite workpiece;
Fig. 21 is a flow diagram of an example of the optimizing of the design data of Fig. 20;
Fig. 22, in combination with Figs. 17, 18 and 19, is a flow diagram of another example of a method for layup and analysis of composite material during fabrication of a composite workpiece;
Fig. 23 is a flow diagram of yet another example of a method for layup and analysis of composite material during fabrication of a composite workpiece;
Fig. 24 is a flow diagram of an example of a method for optimizing design data during fabrication of a composite workpiece;
Fig. 25 is a block diagram of aircraft production and service methodology that implements one or more of the examples of methods for layup and analysis of composite material during fabrication of a composite workpiece disclosed herein; and
Fig. 26 is a schematic illustration of an aircraft that incorporates components fabricated using one or more of the examples of methods for layup and analysis of composite material during fabrication of a composite workpiece disclosed herein.

### DETAILED DESCRIPTION

The various examples of methods 300, 400, 500, 600, 800, 1700, 1800, 1900, 2000, 2200, 2300 for layup and analysis of composite material 902 during fabrication of a composite workpiece 200 disclosed herein provide techniques for in-process analysis and control during the fabrication. The various examples of automated systems 100, 900 for layup and analysis of composite material 902 during fabrication of a composite workpiece 200 disclosed herein enable the methods 300, 400, 500, 600, 800, 1700, 1800, 1900, 2000, 2200, 2300 and techniques for in-process analysis and control during the fabrication. The various examples of methods 2400 for optimizing design data 926 during fabrication of a composite workpiece 200 provide techniques for optimization of design data for the workpiece during the fabrication.

The various techniques for layup and analysis of composite materials during fabrication of composite workpieces disclosed herein may be implemented, for example, to address automated composite lamination fill ratio risk analysis during the design of numerical control programs and lamination fill ratio risk analysis applied during the fabrication for monitoring and process control.

The various methods and automated systems allow rapid analysis and inspection of specific engineering requirements relevant to lamination of composite materials. Particularly, the risk in failing to meet fiber volume fill due to either relative placement of ply, course or tow boundaries or the converges of the ply, course or tow boundaries defined by a numerical control program. The various methods and automated systems also enable an optimization of a numerical control program that specifies ply, course or tow placement to minimize risk associated with meeting fiber volume fill requirements. Additionally, the various methods and automated systems enable the risk analysis for fiber volume fill to be discretized into zones within a lamination surface of the workpiece, such as a surface of a part or subsection within a part. The various methods and automated systems also enable these analyses to be accomplished through the thickness of the part, subsection within a part or more generally multiple layers of a surface.

An additional feature of the various methods and automated systems is that they allow alternate methods of in-process measurement, comparative analysis of as-built to as-planned fill ratio and refinement of as-planned requirements to drive manual optimization of numerical control programming. This approach can be modified if a sampling plan is included, whereby a sampling plan maybe refined to meet requirements.

The various methods and automated systems require the input of relevant data sets associated with design, numerical control programming, and engineering requirements. The various methods and automated systems include application programs that generates gridded data structure registering data and separate gridded data structure for analysis and requirements capture. The various methods and automated systems include features for aggregating relevant data in the gridded structure for analysis and enabling through thickness analysis. The various automated systems include a risk analysis module for meeting the specific fiber volume. The various methods include the process to evaluate risk analysis by zone and manually optimize numerical control program design to minimize risk. The various methods and automated systems also include an option for automated optimization of numerical control path start points to optimize numerical control program design.

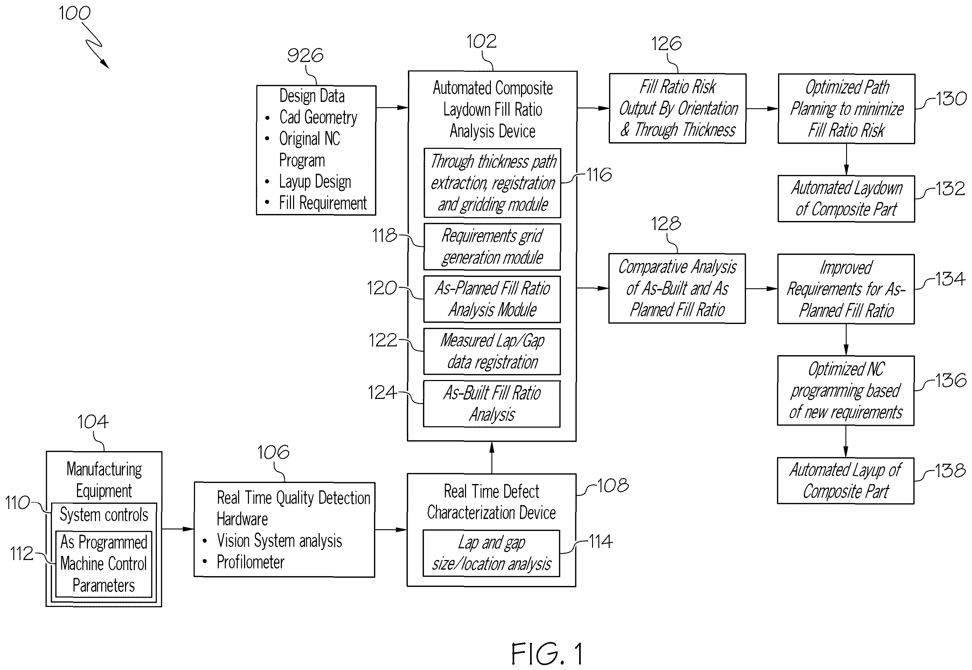

With reference to Fig. 1, in one or more examples an automated system 100 for layup and analysis of composite material 902 during fabrication of a composite workpiece 200 includes design data 926, an automated composite laydown fill ratio analysis device 102, manufacturing equipment 104, real time quality detection hardware 106 and a real time defect characterization device 108. The design data 926 is in operative communication with the automated composite laydown fill ratio analysis device 102. The design data 926, for example, includes a computer aided design geometry, an original numerical control program, a layup design and a fill requirement. The manufacturing equipment 104 is in operative communication with the real time quality detection hardware 106. The manufacturing equipment 104 includes system controls 110 with programmed machine control parameters 112. The real time quality detection hardware 106 is in operative communication with the real time defect characterization device 108. The real time quality detection hardware 106, for example, includes vision system analysis and a profilometer. The real time defect characterization device 108 is in operative communication with the automated composite laydown fill ratio analysis device 102. The real time defect characterization device 108 includes lap and gap size/location analysis 114.

The automated composite laydown fill ratio analysis device 102 includes a through thickness path extraction, registration and gridding module 116, a requirements grid generation module 118, an as-planned fill ratio analysis module 120, measured lap/gap data registration 122 and as-build fill ratio analysis 124. The automated composite laydown fill ratio analysis device 102 is in operative communication with a fill ratio risk output by orientation and through thickness 126. The automated composite laydown fill ratio analysis device 102 is also in operative communication with a comparative analysis of as-built and as-planned fill ratio 128.

The fill ratio risk output by orientation and through thickness 126 is in operative communication with an optimized path planning to minimize fill ratio risk 130. The optimized path planning to minimize fill ratio risk 130 is in operative communication with an automated laydown of composite part 132.

The comparative analysis of as-built and as-planned fill ratio 128 is in operative communication with an improved requirements for as-planned fill ratio 134. The improved requirements for an as-planned fill ratio 134 is in operative communication with an optimized numerical control programming based of new requirements 136. The optimized numerical control programming based of new requirements 136 is in operative communication with an automated layup of composite part 138.

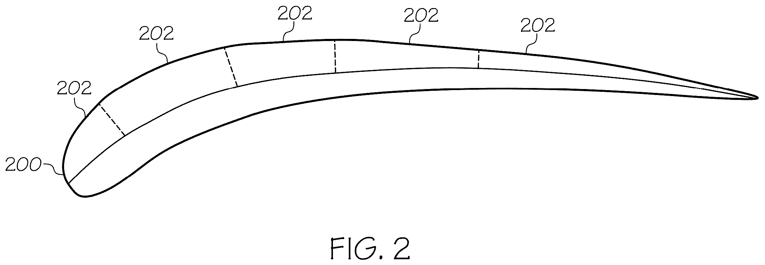

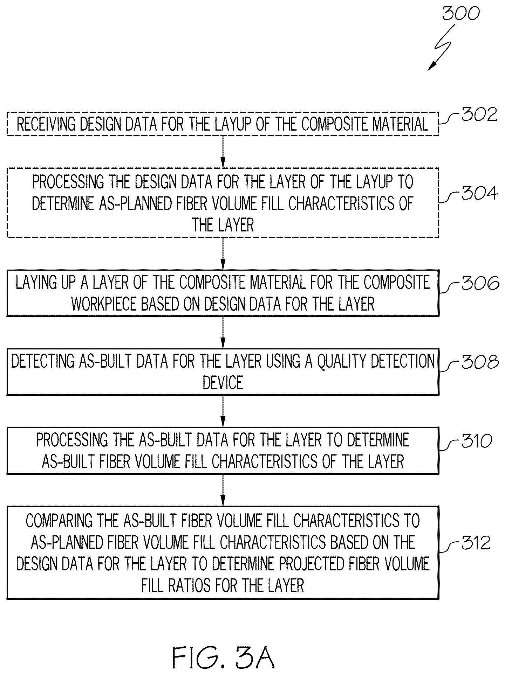

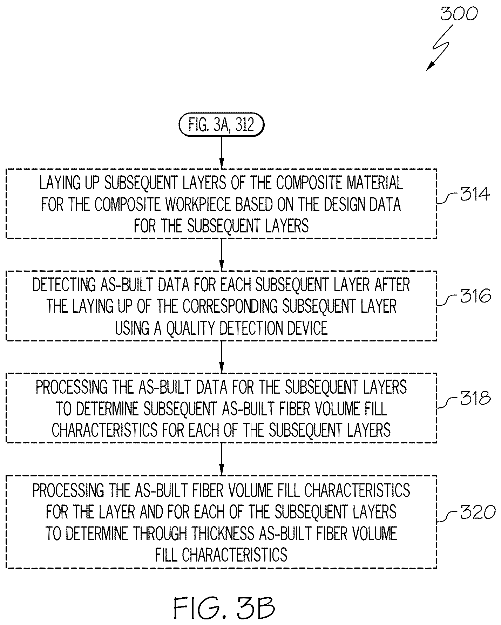

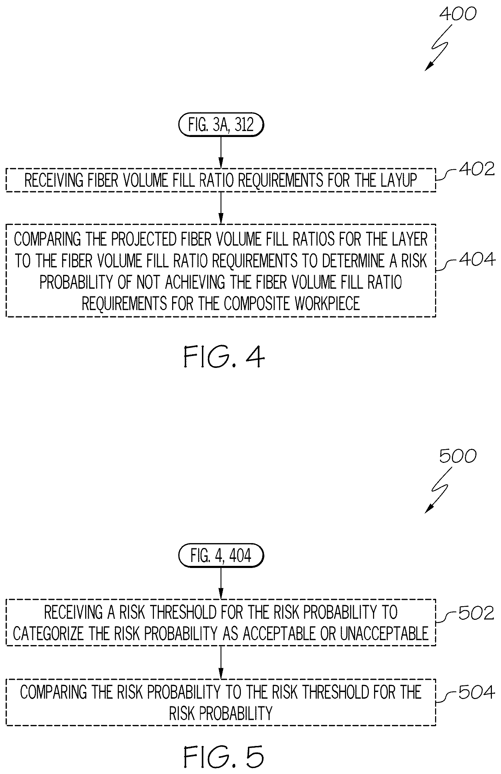

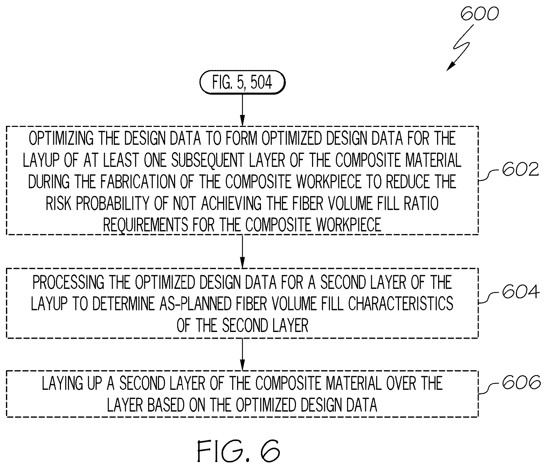

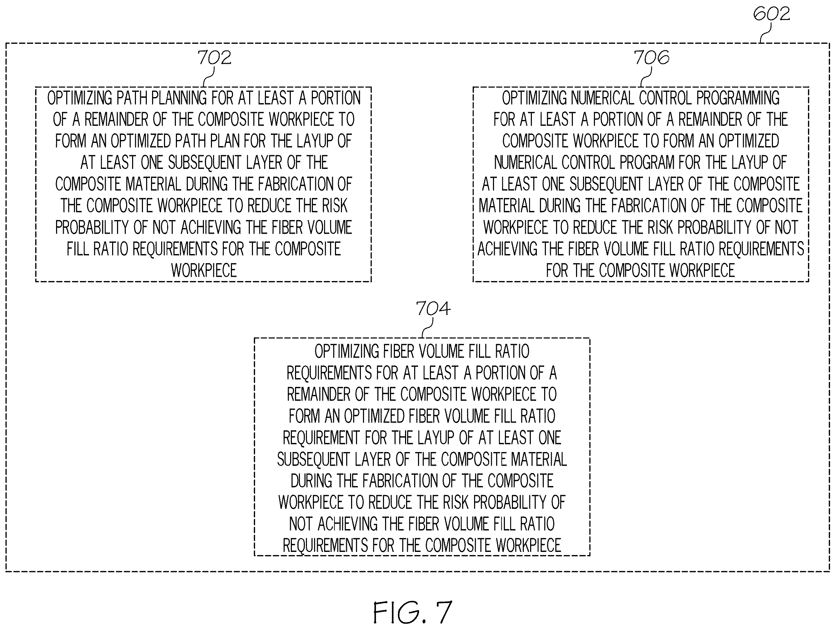

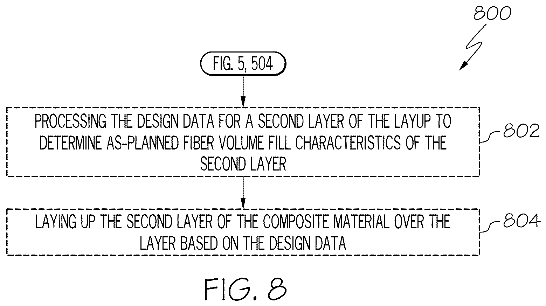

Referring generally to Figs 2, 3A-B, 4-10, 11A-C, 12A-B and 14-16, by way of examples, the present disclosure is directed to methods 300, 400, 500, 600, 800 for layup and analysis of composite materials 902 during fabrication of composite workpieces 200. Fig. 2 provides an example of a composite workpiece 200. Figs. 3A and 3B provide examples of the method 300 for layup and analysis of composite material 902 during fabrication of a composite workpiece 200. Fig. 4, in combination with Fig. 3A, provides an example of the method 400 for layup and analysis of composite material 902 during fabrication of a composite workpiece 200. Fig. 5, in combination with Figs. 3A and 4, provides an example of the method 500 for layup and analysis of composite material 902 during fabrication of a composite workpiece 200. Fig. 6, in combination with Figs. 3A, 4 and 5, provides an example of the method 600 for layup and analysis of composite material 902 during fabrication of a composite workpiece 200. Fig. 7 provides examples of the optimizing 602 of the design data 926 of Fig. 6. Fig. 8, in combination with Figs. 3A, 4 and 5, provides an example of the method 800 for layup and analysis of composite material 902 during fabrication of a composite workpiece 200.

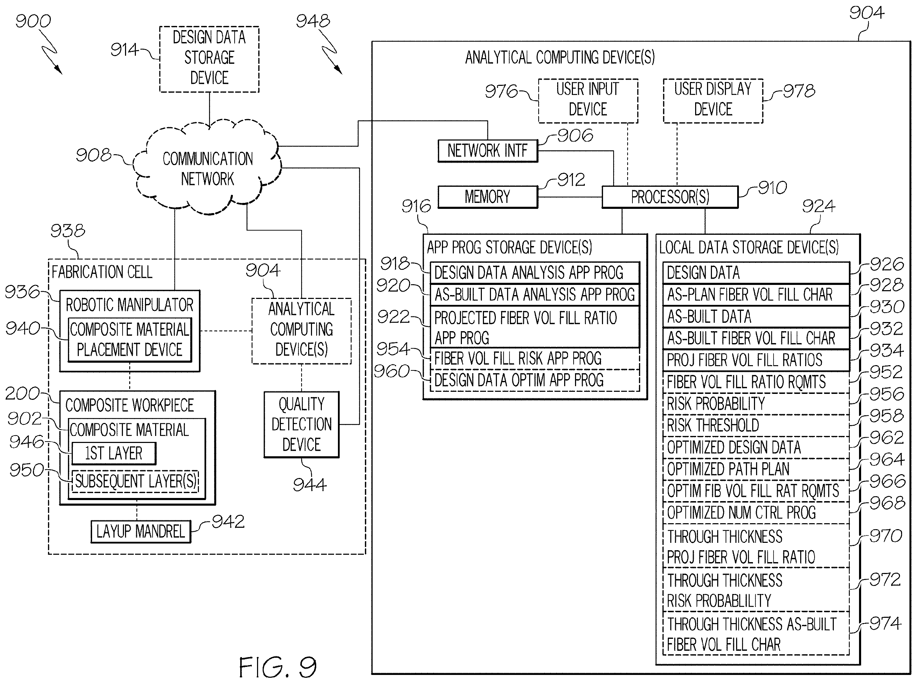

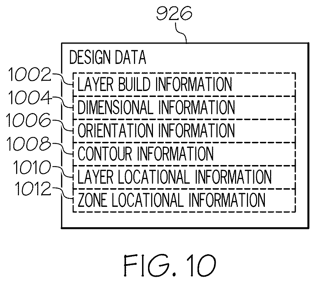

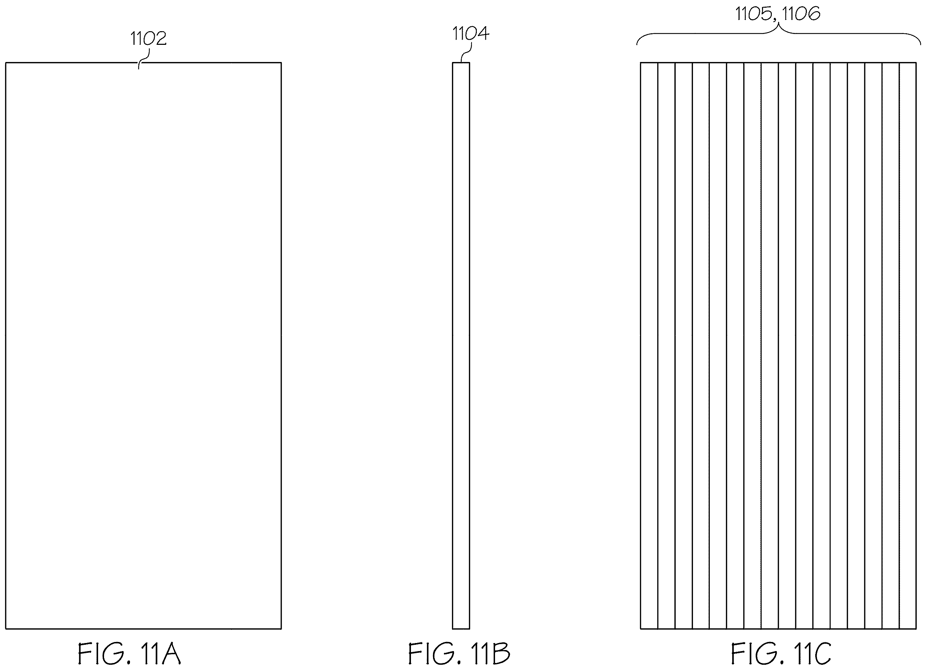

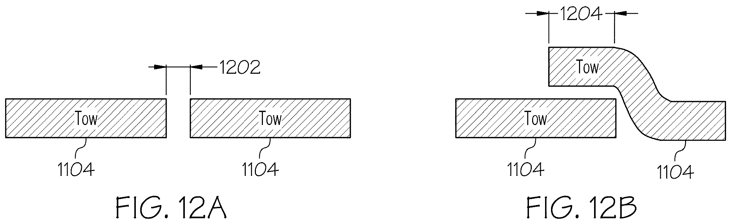

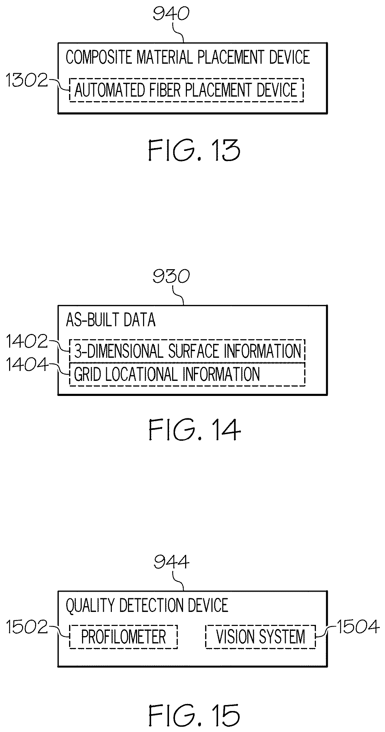

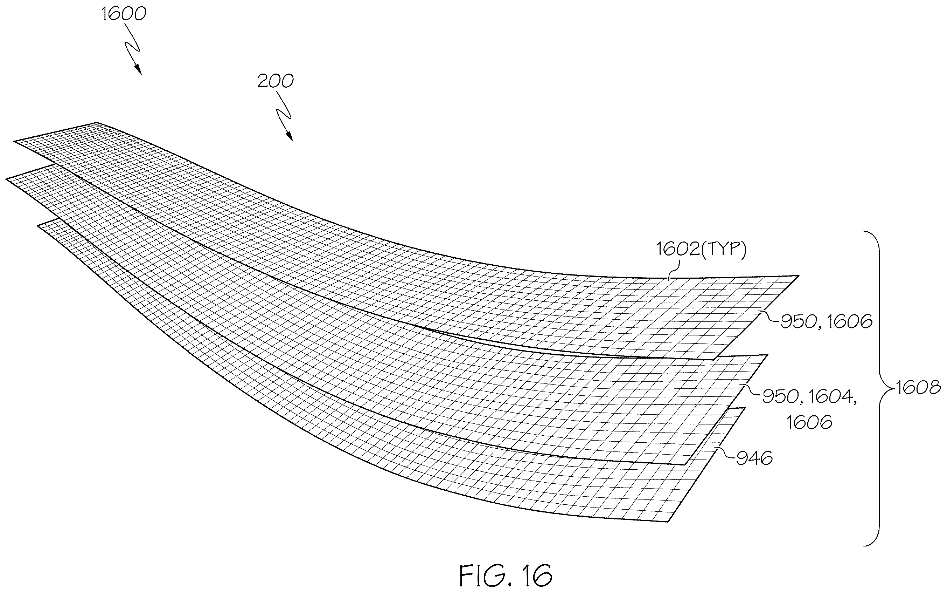

Fig. 9 is a functional block diagram of an example of an automated system 900 for layup and analysis of composite material 902 during fabrication of a composite workpiece 200. Fig. 10 is a block diagram showing examples of design data 926 used in conjunction with layup and analysis of composite material 902 during fabrication of a composite workpiece 200. Figs. 11A-11C are functional diagrams showing examples of plies 1102, tows 1105 and courses 1106 of tows 1105 used in conjunction with layup of composite material 902 during fabrication of a composite workpiece 200. Figs. 12A and 12B are functional diagrams showing examples of gaps 1202 and overlaps 1204 between adjacent tows 1105 in conjunction with layup and analysis of composite material 902 during fabrication of a composite workpiece 200. Fig. 14 is a block diagram showing examples of as-built data 930 used in conjunction with layup and analysis of composite material 902 during fabrication of a composite workpiece 200. Fig. 15 is a block diagram showing examples of the quality detection device 944 of Fig. 9. Fig. 16 is a functional diagram showing an example of a grid structure 1600 for a composite workpiece 200 used in conjunction with layup and analysis of composite material 902 during fabrication of a composite workpiece 200.

With reference again to Figs. 2, 3A-B, 9, 10, 11A-C, 12A-B and 14-16, in one or more examples, a method 300 (see Fig. 3A-B) for layup and analysis of composite materials 902 during fabrication of composite workpieces 200 includes laying up 306 a layer 946 of the composite material 902 for the composite workpiece 200 based on design data 926 for the layer 946. At 308, as-built data 930 for the layer 946 is detected using a quality detection device 944. At 310, the as-built data 930 for the layer 946 is processed to determine as-built fiber volume fill characteristics 932 of the layer 946. At 312, the as-built fiber volume fill characteristics 932 are compared to as-planned fiber volume fill characteristics 928 based on the design data 926 for the layer 946 to determine projected fiber volume fill ratios 934 for the layer 946.

In some examples of the method 300, the layer 946 of the composite material 902 is laid up on a layup mandrel 942 in a fabrication cell 938. A second layer 1604 of the composite material 902 is laid up over the layer 946 and subsequent layers 950 of the composite material 902 are laid up over current layers 1608. In some examples of the method 300, the detecting 308 of the as-built data 930 is performed while the layer 946 is on a layup mandrel 942. In some examples, the method 300 continues from 312 to 314. At 314, subsequent layers 950 of the composite material 902 for the composite workpiece 200 are laid up based on the design data 926 for the subsequent layers 950. At 316, as-built data 930 is detected for each subsequent layer 950 after the laying up 314 of the corresponding subsequent layer 950 using a quality detection device 944. At 318, the as-built data 930 for the subsequent layers 950 is processed to determine subsequent as-built fiber volume fill characteristics 932 for each of the subsequent layers 950. At 320, the as-built fiber volume fill characteristics 932 is processed for the layer 946 and for each of the subsequent layers 950 to determine through thickness as-built fiber volume fill characteristics 974.

In some examples of the method 300, the composite material 902 includes a reinforcement material and a matrix material. In some examples, the reinforcement material includes fiber materials, and the matrix material includes resin material. In some examples, the fiber materials include unidirectional fiber materials. In some examples of the method 300, the design data 926 for the layup includes layer build information 1002. In some examples of the method 300, the design data 926 for the layup includes dimensional information 1004 for the composite material 902, orientation information 1006 for the composite material 902, dimensional information 1004 for the composite workpiece 200, contour information 1008 for the composite workpiece 200 or any other suitable design data for the layup in any suitable combination.

In some examples of the method 300, the composite workpiece 200 includes multiple layers 946, 950 and the design data 926 for the layup includes layer locational information 1010 for at least a portion of the design data 926. In some examples of the method 300, the composite workpiece 200 includes multiple zones 202 and the design data 926 for the layup includes zone locational information 1012 for at least a portion of the design data 926. In some examples of the method 300, the layer 946 includes at least one of a ply 1102 of composite material 902, a plurality of tows 1105 of composite material 902, and a plurality of courses 1106 of tows 1105 of composite material 902. In some examples of the method 300, the as-planned fiber volume fill characteristics 928 take into account planned overlaps 1204 and planned gaps 1202 for each layer 946, 950 of the composite material 902 in the composite workpiece 200. In some examples of the method 300, the as-built data 930 for the layer 946 includes three-dimensional surface information 1402 representative of a surface of the layer 946 to capture actual overlaps 1204 and actual gaps 1202 for the layer 946 of the composite material 902. In some examples of the method 300, the quality detection device 944 includes a profilometer 1502, a vision system 1504 or any other suitable quality detection device in any suitable combination.

In some examples of the method 300, the as-built data 930 is divided into a grid structure 1600 for the composite workpiece 200 that includes a plurality of data cells 1602 for each layer 946, 950 of the composite workpiece 200 and the as-built data 930 includes grid locational information 1404. In some examples of the method 300, the as-built fiber volume fill characteristics 932 for the layer 946 take into account measured overlaps 1204 and measured gaps 1202 for the layer 946 of the composite material 902 in the composite workpiece 200. In some examples, the method 300 also includes receiving 302 design data 926 for the layup of the composite material 902. At 304, the design data 926 for the layer 946 of the layup is processed to determine as-planned fiber volume fill characteristics 928 of the layer 946. In this example, the method continues from 304 to 306.

With reference again to Figs. 2, 3A, 4, 9 and 10, in one or more examples, a method 400 (see Fig. 4) for layup and analysis of composite materials 902 during fabrication of composite workpieces 200 includes the method 300 of Fig. 3A and continues from 312 to 402 where fiber volume fill ratio requirements 952 for the layup are received. At 404, the projected fiber volume fill ratios 934 for the layer 946 is compared to the fiber volume fill ratio requirements 952 to determine a risk probability 956 of not achieving the fiber volume fill ratio requirements 952 for the composite workpiece 200. In some examples of the method 400, the composite workpiece 200 includes multiple layers 946, 950 and the fiber volume fill ratio requirements 952 for the layup includes layer locational information 1010 for each layer 946, 950 of the composite workpiece 200 individually and for the multiple layers 946, 950 collectively.

With reference again to Figs. 3A, 4, 5 and 9, in one or more examples, a method 500 (see Fig. 5) for layup and analysis of composite materials 902 during fabrication of composite workpieces 200 includes the method 300 of Fig. 3A and the method 400 of Fig. 4. The method 500 continues from 404 of Fig. 4 to 502 where a risk threshold 958 for the risk probability 956 is received to categorize the risk probability 956 as acceptable or unacceptable. At 504, the risk probability 956 is compared to the risk threshold 958 for the risk probability 956.

With reference again to Figs. 2, 3A, 4-7 and 9, in one or more examples, where the risk probability 956 exceeds the risk threshold 958, the risk probability 956 is unacceptable and a method 600 (see Fig. 6) for layup and analysis of composite materials 902 during fabrication of composite workpieces 200 includes the method 300 of Fig. 3A, the method 400 of Fig. 4 and the method 500 of Fig. 5. The method 600 continues from 504 of Fig. 5 to 602 where the design data 926 is optimized to form optimized design data 962 for the layup of at least one subsequent layer 950 of the composite material 902 during the fabrication of the composite workpiece 200 to reduce the risk probability 956 of not achieving the fiber volume fill ratio requirements 952 for the composite workpiece 200. At 604, the optimized design data 962 for a second layer 1604 of the layup is processed to determine as-planned fiber volume fill characteristics 928 of the second layer 1604. At 606, a second layer 1604 of the composite material 902 is laid up over the layer 946 based on the optimized design data 962.

In some examples of the method 600, the optimizing 602 of the design data 926 includes optimizing 702 (see Fig. 7) path planning for at least a portion of a remainder of the composite workpiece 200 to form an optimized path plan 964 for the layup of at least one subsequent layer 950 of the composite material 902 during the fabrication of the composite workpiece 200 to reduce the risk probability 956 of not achieving the fiber volume fill ratio requirements 952 for the composite workpiece 200. **In** some examples of the method 600, the optimizing 602 of the design data 926 includes optimizing 704 fiber volume fill ratio requirements 952 for at least a portion of a remainder of the composite workpiece 200 to form an optimized fiber volume fill ratio requirement 966 for the layup of at least one subsequent layer 950 of the composite material 902 during the fabrication of the composite workpiece 200 to reduce the risk probability 956 of not achieving the fiber volume fill ratio requirements 952 for the composite workpiece 200. **In** some examples of the method 600, the optimizing 602 of the design data 926 includes optimizing 706 numerical control programming for at least a portion of a remainder of the composite workpiece 200 to form an optimized numerical control program 968 for the layup of at least one subsequent layer 950 of the composite material 902 during the fabrication of the composite workpiece 200 to reduce the risk probability 956 of not achieving the fiber volume fill ratio requirements 952 for the composite workpiece 200.

With reference again to Figs. 3A, 4, 5, 8, 9 and 16, in one or more examples, where the risk probability 956 does not exceed the risk threshold 958, the risk probability 956 is acceptable and a method 800 (see Fig. 8) for layup and analysis of composite materials 902 during fabrication of composite workpieces 200 includes the method 300 of Fig. 3A, the method 400 of Fig. 4 and the method 500 of Fig. 5. The method 800 continues from 504 of Fig. 5 to 802 where the design data 926 for a second layer 1604 of the layup is processed to determine as-planned fiber volume fill characteristics 928 of the second layer 1604. At 804, the second layer 1604 of the composite material 902 is laid up over the layer 946 based on the design data 926.

Referring generally to Figs. 2, 9, 10, 11A-C, 12A-B and 13-16, by way of examples, the present disclosure is directed to an automated system 900 for layup and analysis of composite materials 902 during fabrication of composite workpieces 200. Fig. 2 provides an example of a composite workpiece 200. Fig. 9 is a functional block diagram of an example of the automated system 900 for layup and analysis of composite material 902 during fabrication of a composite workpiece 200. Fig. 10 is a block diagram showing examples of design data 926 used in conjunction with layup and analysis of composite material 902 during fabrication of a composite workpiece 200. Figs. 11A-11C are functional diagrams showing examples of plies 1102, tows 1105 and courses 1106 of tows 1105 used in conjunction with layup of composite material 902 during fabrication of a composite workpiece 200. Figs. 12A and 12B are functional diagrams showing examples of gaps 1202 and overlaps 1204 between adjacent tows 1105 in conjunction with layup and analysis of composite material 902 during fabrication of a composite workpiece 200. Fig. 13 is a block diagram showing an example of the composite material placement device 940 of Fig. 9. Fig. 14 is a block diagram showing examples of as-built data 930 used in conjunction with layup and analysis of composite material 902 during fabrication of a composite workpiece 200. Fig. 15 is a block diagram showing examples of the quality detection device 944 of Fig. 9. Fig. 16 is a functional diagram showing an example of a grid structure 1600 for a composite workpiece 200 used in conjunction with layup and analysis of composite material 902 during fabrication of a composite workpiece 200.

With reference again to Figs. 2, 9, 10, 11A-C, 12A-B and 13-16, in one or more examples, an automated system 900 for layup and analysis of composite material 902 during fabrication of a composite workpiece 200 is disclosed. The automated system 900 includes at least one analytical computing device 904, a robotic manipulator 936, a layup mandrel 942 and a quality detection device 944. The at least one analytical computing device 904 storing a design data analysis application program 918, an as-built data analysis application program 920 and a projected fiber volume fill ratio application program 922. The robotic manipulator 936 in a fabrication cell 938 and in operative communication with the at least one analytical computing device 904. The robotic manipulator 936 including a composite material placement device 940. The layup mandrel 942 in the fabrication cell 938 and configured for layup of the composite material 902. The quality detection device 944 in the fabrication cell 938 and in operative communication with the at least one analytical computing device 904. The at least one analytical computing device 904 may also include a user input device 976 and a user display device 978.

In some examples of the automated system 900, the at least one analytical computing device 904 includes a network interface 906, at least one processor 910 and associated memory 912, at least one application program storage device 916 and at least one local data storage device 924. The network interface 906 in operative communication with a communication network 908. The at least one processor 910 in operative communication with a design data storage device 914 via the network interface 906 and the communication network 908. The at least one application program storage device 916 in operative communication with the at least one processor 910 and storing the design data analysis application program 918, the as-built data analysis application program 920 and the projected fiber volume fill ratio application program 922. The at least one application program storage device 916 also storing one or more of a fiber volume fill risk application program 954 and a design data optimization application program 960. The at least one local data storage device 924 configured to at least temporarily store design data 926, as-planned fiber volume fill characteristics 928, as-built data 930, as-built fiber volume fill characteristics 932 and projected fiber volume fill ratios 934. The at least one local data storage device 924 also configured to at least temporarily store one or more of fiber volume fill ratio requirements, risk probability, risk threshold, optimized path plan, optimized fiber volume fill ratio requirements and optimized numerical control program.

In some examples, the design data storage device 914 is configured to store the design data 926, design requirements and validation records for the composite workpiece 200. In some examples, the design data 926 for the layup includes layer build information 1002. In some examples, design data 926 for the layup includes dimensional information 1004 for the composite material 902, orientation information 1006 for the composite material 902, dimensional information 1004 for the composite workpiece 200, contour information 1008 for the composite workpiece 200 or any other suitable design data for the layup in any suitable combination. In some examples, the composite workpiece 200 includes multiple layers 946, 950 and the design data 926 for the layup includes layer locational information 1010 for at least a portion of the design data 926. In some examples, the composite workpiece 200 includes multiple zones 202 and the design data 926 for the layup includes zone locational information 1012 for at least a portion of the design data 926.

In some examples of the automated system 900, the at least one analytical computing device 904 includes one or more analytical computing device 904 located in a remote location 948 in relation to the fabrication cell 938, one or more analytical computing device 904 located within the fabrication cell 938 or any suitable combination thereof. In some examples of the automated system 900, the composite material placement device 940 includes an automated fiber placement device 1302. In some examples, the automated fiber placement device 1302 is configured to lay at least one tow 1104 on the layup mandrel 942. In some examples, the automated fiber placement device 1302 is configured to lay a course 1106 of tows 1105 on the layup mandrel 942. In some examples of the automated system 900, the quality detection device 944 includes a profilometer 1502, a vision system 1504 or any other suitable quality detection device in any suitable combination.

In some examples of the automated system 900, the robotic manipulator 936 and the composite material placement device 940 are configured to layup a layer 946 of the composite material 902 for the composite workpiece 200 on the layup mandrel 942 in the fabrication cell 938 based on design data 926 for the layer 946. In this and certain other examples, after the layup of the layer 946, the quality detection device 944 is configured to detect as-built data 930 for the layer 946 on the layup mandrel 942. The at least one analytical computing device 904, in conjunction with running the as-built data analysis application program 920, is configured to process the as-built data 930 for the layer 946 to determine as-built fiber volume fill characteristics 932 of the layer 946 on the layup mandrel 942. The at least one analytical computing device 904, in conjunction with running the projected fiber volume fill ratio application program 922, is configured to compare the as-built fiber volume fill characteristics 932 to as-planned fiber volume fill characteristics 928 based on the design data 926 for the layer 946 to determine projected fiber volume fill ratios 934 for the layer 946.

In some examples, the layer 946 includes at least one of a ply 1102 of composite material 902, a plurality of tows 1105 of composite material 902, and a plurality of courses 1106 of tows 1105 of composite material 902. In some examples, the as-planned fiber volume fill characteristics 928 take into account planned overlaps 1204 and planned gaps 1202 for each layer 946, 950 of the composite material 902 in the composite workpiece 200. In some examples, the as-built data 930 is divided into a grid structure 1600 for the composite workpiece 200 that includes a plurality of data cells 1602 for each layer 946, 950 of the composite workpiece 200 and the as-built data 930 includes grid locational information 1404. In some examples, the as-built data 930 for the layer 946 includes three-dimensional surface information 1402 representative of a surface of the layer 946 to capture actual overlaps 1204 and actual gaps 1202 for the layer 946 of the composite material 902. In some examples, the as-built fiber volume fill characteristics 932 for the layer 946 take into account measured overlaps 1204 and measured gaps 1202 for the layer 946 of the composite material 902 in the composite workpiece 200.

In some examples, the at least one analytical computing device 904 is configured to receive design data 926 for the layup of the composite material 902 from a design data storage device 914 via a network interface 906 and a communication network 908. In this and certain other examples, the at least one analytical computing device 904, in conjunction with running the design data analysis application program 918, is configured to process the design data 926 for the layer 946 of the layup to determine as-planned fiber volume fill characteristics 928 of the layer 946.

In some examples, the at least one analytical computing device 904 is configured to receive fiber volume fill ratio requirements 952 for the layup from a design data storage device 914 via a network interface 906 and a communication network 908. In this and certain other examples, the at least one analytical computing device 904, in conjunction with running the fiber volume fill risk application program 954, is configured to compare the projected fiber volume fill ratios 934 for the layer 946 to the fiber volume fill ratio requirements 952 to determine a risk probability 956 of not achieving the fiber volume fill ratio requirements 952 for the composite workpiece 200 after the layup of the layer 946.

In some examples, the at least one analytical computing device 904 is configured to receive a risk threshold 958 for the risk probability 956 to categorize the risk probability 956 as acceptable or unacceptable from the design data storage device 914 via a network interface 906 and a communication network 908. In this and certain other examples, the at least one analytical computing device 904, in conjunction with running the fiber volume fill risk application program 954, is configured to compare the risk probability 956 after the layup of the layer 946 to the risk threshold 958 for the risk probability 956.

In some examples, where the risk probability 956 exceeds the risk threshold 958, the risk probability 956 is unacceptable and the at least one analytical computing device 904, in conjunction with running a design data optimization application program 960, is configured to optimize the design data 926 to form optimized design data 962 for the layup of at least one subsequent layer 950 of the composite material 902 during the fabrication of the composite workpiece 200 to reduce the risk probability 956 of not achieving the fiber volume fill ratio requirements 952 for the composite workpiece 200 after the layup of the at least one subsequent layer 950. In this and certain other examples, the at least one analytical computing device 904, in conjunction with running the design data analysis application program 918, is configured to process the optimized design data 962 for a second layer 1604 of the layup to determine as-planned fiber volume fill characteristics 928 of the second layer 1604. The robotic manipulator 936 and the composite material placement device 940 are configured to layup a second layer 1604 of the composite material 902 over the layer 946 on the layup mandrel 942 based on the optimized design data 962.

In some examples, the at least one analytical computing device 904, in conjunction with running the design data optimization application program 960, is configured to optimize path planning for at least a portion of a remainder of the composite workpiece 200 to form an optimized path plan 964 for the layup of at least one subsequent layer 950 of the composite material 902 during the fabrication of the composite workpiece 200 to reduce the risk probability 956 of not achieving the fiber volume fill ratio requirements 952 for the composite workpiece 200 after the layup of the at least one subsequent layer 950.

In some examples, the at least one analytical computing device 904, in conjunction with running the design data optimization application program 960, is configured to optimize fiber volume fill ratio requirements 952 for at least a portion of a remainder of the composite workpiece 200 to form an optimized fiber volume fill ratio requirement 966 for the layup of at least one subsequent layer 950 of the composite material 902 during the fabrication of the composite workpiece 200 to reduce the risk probability 956 of not achieving the fiber volume fill ratio requirements 952 for the composite workpiece 200 after the layup of the at least one subsequent layer 950.

In some examples, the at least one analytical computing device 904, in conjunction with running the design data optimization application program 960, is configured to optimize numerical control programming for at least a portion of a remainder of the composite workpiece 200 to form an optimized numerical control program 968 for the layup of at least one subsequent layer 950 of the composite material 902 during the fabrication of the composite workpiece 200 to reduce the risk probability 956 of not achieving the fiber volume fill ratio requirements 952 for the composite workpiece 200 after the layup of the at least one subsequent layer 950.

In some examples, where the risk probability 956 does not exceed the risk threshold 958, the risk probability 956 is acceptable and the at least one analytical computing device 904, in conjunction with running the design data analysis application program 918, is configured to process the design data 926 for a second layer 1604 of the layup to determine as-planned fiber volume fill characteristics 928 of the second layer 1604. In this and certain other examples, the robotic manipulator 936 and the composite material placement device 940 are configured to layup the second layer 1604 of the composite material 902 over the layer 946 on the layup mandrel 942 based on the design data 926.

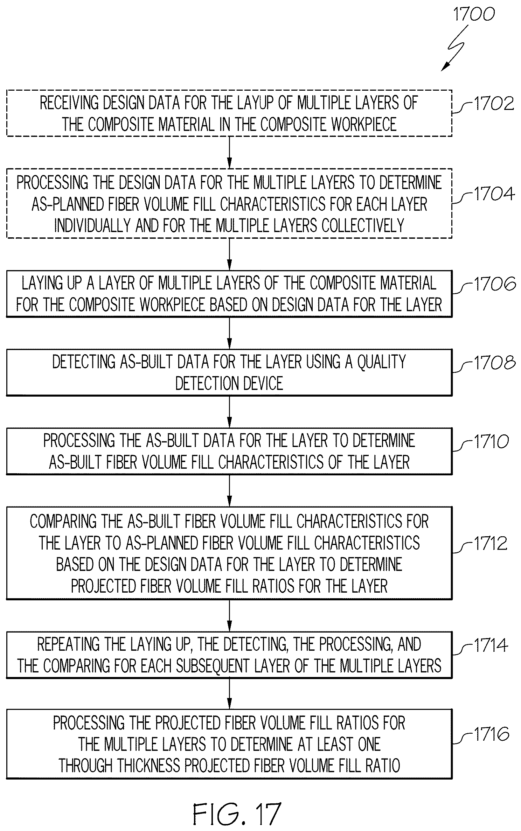

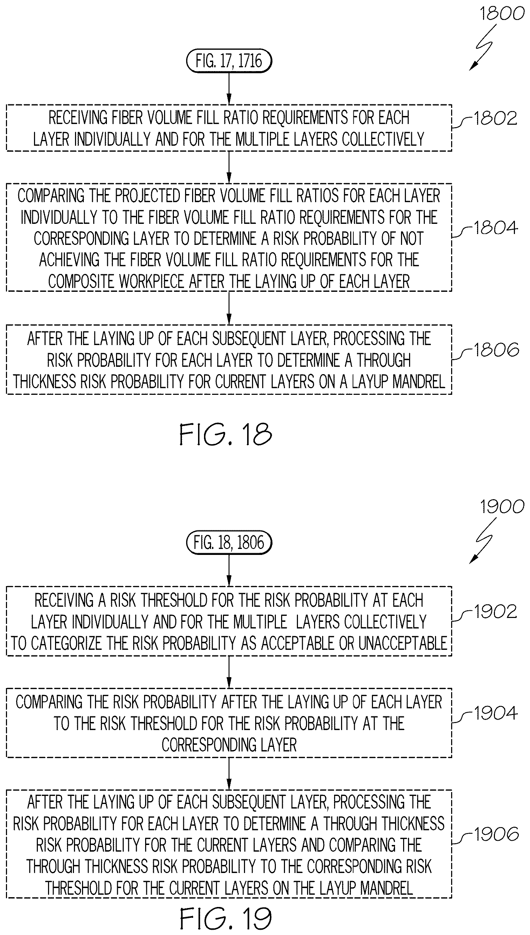

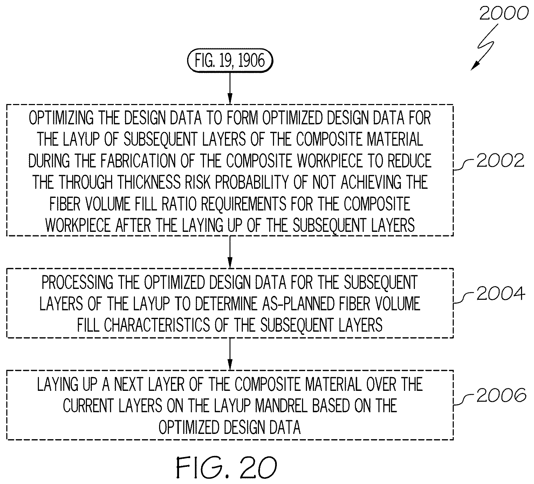

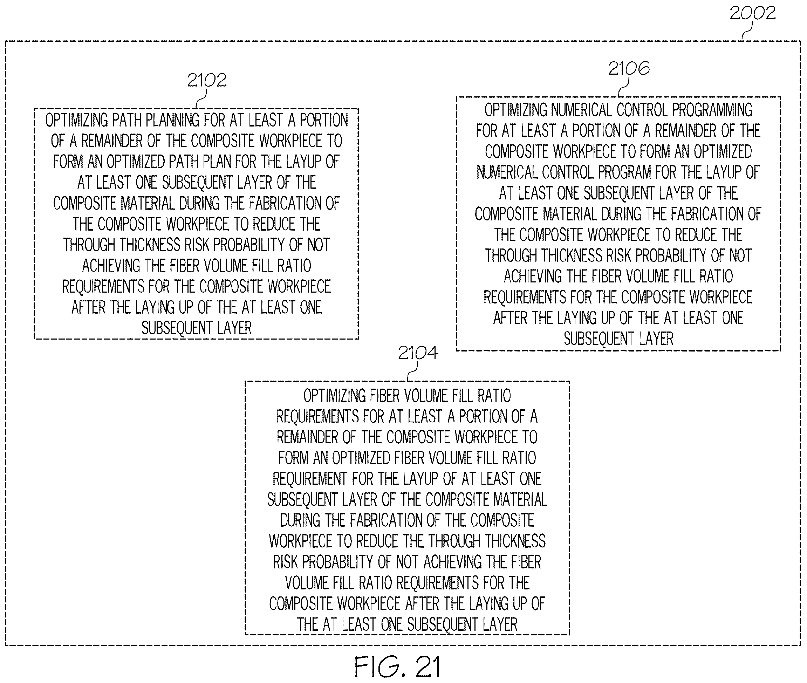

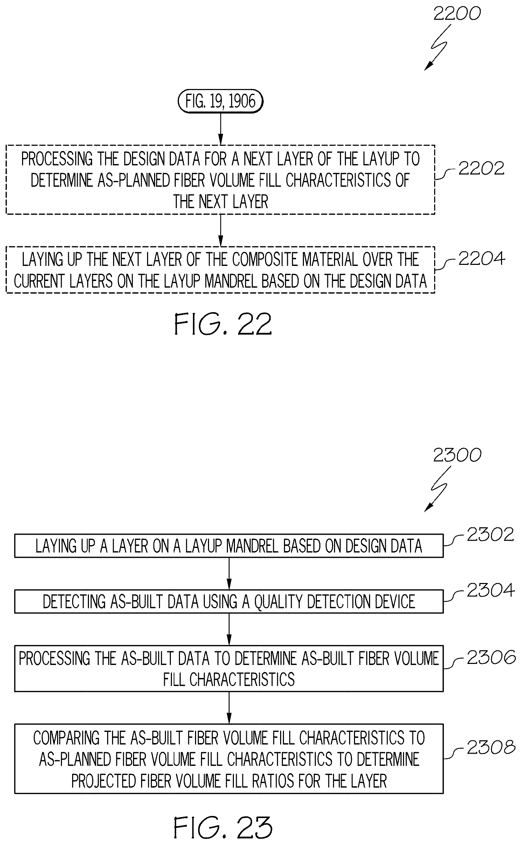

Referring generally to Figs. 2, 9, 10, 11A-C, 12A-B, 14 and 16-22, by way of examples, the present disclosure is directed to methods 1700, 1800, 1900, 2000, 2200 for layup and analysis of composite materials 902 during fabrication of composite workpieces 200. Fig. 2 provides an example of a composite workpiece 200. Fig. 9 is a functional block diagram of an example of an automated system 900 for layup and analysis of composite material 902 during fabrication of a composite workpiece 200. Fig. 10 is a block diagram showing examples of design data 926 used in conjunction with layup and analysis of composite material 902 during fabrication of a composite workpiece 200. Figs. 11A-11C are functional diagrams showing examples of plies 1102, tows 1105 and courses 1106 of tows 1105 used in conjunction with layup of composite material 902 during fabrication of a composite workpiece 200. Figs. 12A and 12B are functional diagrams showing examples of gaps 1202 and overlaps 1204 between adjacent tows 1105 in conjunction with layup and analysis of composite material 902 during fabrication of a composite workpiece 200. Fig. 14 is a block diagram showing examples of as-built data 930 used in conjunction with layup and analysis of composite material 902 during fabrication of a composite workpiece 200. Fig. 16 is a functional diagram showing an example of a grid structure 1600 for a composite workpiece 200 used in conjunction with layup and analysis of composite material 902 during fabrication of a composite workpiece 200. Fig. 17 provides an example of the method 1700 for layup and analysis of composite material 902 during fabrication of a composite workpiece 200. Fig. 18, in combination with Fig. 17, provides an example of the method 1800 for layup and analysis of composite material 902 during fabrication of a composite workpiece 200. Fig. 19, in combination with Figs. 17 and 18, provides an example of the method 1900 for layup and analysis of composite material 902 during fabrication of a composite workpiece 200. Fig. 20, in combination with Figs. 17, 18 and 19, provides an example of the method 2000 for layup and analysis of composite material 902 during fabrication of a composite workpiece 200. Fig. 21 provides examples of the optimizing 2002 of the design data 926 of Fig. 20. Fig. 22, in combination with Figs. 17, 18 and 19, provides an example of the method 2200 for layup and analysis of composite material 902 during fabrication of a composite workpiece 200.

With reference again to Figs. 2, 9, 10, 11A-C, 12A-B, 14, 16 and 17, in one or more examples, a method 1700 (see Fig. 17) for layup and analysis of composite material 902 during fabrication of a composite workpiece 200 includes laying up 1706 a layer 946 of multiple layers 946, 950 of the composite material 902 for the composite workpiece 200 based on design data 926 for the layer 946. At 1708, as-built data 930 for the layer 946 is detected using a quality detection device 944. At 1710, the as-built data 930 for the layer 946 is processed to determine as-built fiber volume fill characteristics 932 of the layer 946. At 1712, the as-built fiber volume fill characteristics 932 for the layer 946 are compared to as-planned fiber volume fill characteristics 928 based on the design data 926 for the layer 946 to determine projected fiber volume fill ratios 934 for the layer 946. At 1714, the detecting 1708, the processing 1710, and the comparing 1712 for each subsequent layer 950 of the multiple layers 946, 950 are repeated. At 1712, the projected fiber volume fill ratios 934 for the multiple layers 946, 950 are processed to determine at least one through thickness projected fiber volume fill ratio 970.

In some examples of the method 1700, the layer 946 of the composite material 902 is laid up on a layup mandrel 942 in a fabrication cell 938. In some examples of the method 1700, the detecting 1708 of the as-built data 930 is performed while the layer 946 is on a layup mandrel 942. In some examples of the method 1700, the design data 926 for the layup includes layer build information 1002. In some examples of the method 1700, the design data 926 for the layup includes dimensional information 1004 for the composite material 902, orientation information 1006 for the composite material 902, dimensional information 1004 for the composite workpiece 200, contour information 1008 for the composite workpiece 200 or any other suitable design data for the layup in any suitable combination. In some examples of the method 1700, the design data 926 for the layup includes layer locational information 1010 for at least a portion of the design data 926. In some examples of the method 1700, the composite workpiece 200 includes multiple zones 202 and the design data 926 for the layup includes zone locational information 1012 for at least a portion of the design data 926. In some examples of the method 1700, each layer 946, 950 of the multiple layers 946, 950 includes at least one of a ply 1102 of composite material 902, a plurality of tows 1105 of composite material 902, and a plurality of courses 1106 of tows 1105 of composite material 902. In some examples of the method 1700, the as-planned fiber volume fill characteristics 928 take into account planned overlaps 1204 and planned gaps 1202 for each layer 946, 950 of the multiple layers 946, 950.

In some examples of the method 1700, the as-built data 930 for each layer 946, 950 of the multiple layers 946, 950 includes three-dimensional surface information 1402 representative of a surface of the corresponding layer 946, 950 to capture actual overlaps 1204 and actual gaps 1202 of the corresponding layer 946, 950. In some examples of the method 1700, the as-built data 930 is divided into a grid structure 1600 for the composite workpiece 200 that includes a plurality of data cells 1602 for each layer 946, 950 of the multiple layers 946, 950 and the as-built data 930 includes grid locational information 1404. In some examples of the method 1700, the as-built fiber volume fill characteristics 932 for each layer 946, 950 of the multiple layers 946, 950 takes into account measured overlaps 1204 and measured gaps 1202 for the corresponding layer 946, 950 of the multiple layers 946, 950. In some examples, method 1700 also includes receiving 1702 design data 926 for the layup of multiple layers 946, 950 of the composite material 902 in the composite workpiece 200. At 1704, the design data 926 for the multiple layers 946, 950 is processed to determine as-planned fiber volume fill characteristics 928 for each layer 946, 950 individually and for the multiple layers 946, 950 collectively.

With reference again to Figs. 9, 17 and 18, in one or more examples, a method 1800 (see Fig. 18) for layup and analysis of composite material 902 during fabrication of a composite workpiece 200 includes the method 1700 of Fig. 17 and continues from 1716 to 1802 where fiber volume fill ratio requirements 952 are received for each layer 946, 950 individually and for the multiple layers 946, 950 collectively. At 1804, the projected fiber volume fill ratios 934 for each layer 946, 950 individually is compared to the fiber volume fill ratio requirements 952 for the corresponding layer 946, 950 to determine a risk probability 956 of not achieving the fiber volume fill ratio requirements 952 for the composite workpiece 200 after the laying up 1706 of each layer 946, 950. At 1806, after the laying up 1706 of each subsequent layer 950, the risk probability 956 for each layer 946, 950 is processed to determine a through thickness risk probability 972 for current layers 1608 on a layup mandrel 942.

With reference again to Figs. 9 and 17-19, in one or more example, a method 1900 (see Fig. 19) for layup and analysis of composite material 902 during fabrication of a composite workpiece 200 includes the method 1700 of Fig. 17 and the method 1800 of Fig. 18 and continues from 1806 to 1902 where a risk threshold 958 for the risk probability 956 at each layer 946, 950 individually and for the multiple layers 946, 950 collectively is received to categorize the risk probability 956 as acceptable or unacceptable. At 1904, the risk probability 956 after the laying up 1706 of each layer 946, 950 is compared to the risk threshold 958 for the risk probability 956 at the corresponding layer 946, 950. At 1906, after the laying up 1706 of each subsequent layer 950, the risk probability 956 for each layer 946, 950 is processed to determine a through thickness risk probability 972 for the current layers 1608 and comparing the through thickness risk probability 972 to the corresponding risk threshold 958 for the current layers 1608 on the layup mandrel 942.

With reference again to Figs. 9 and 17-21, in one or more examples, where the through thickness risk probability 972 for the current layers 1608 on the layup mandrel 942 exceeds the risk threshold 958, the through thickness risk probability 972 is unacceptable and a method 2000 (see Fig. 20) for layup and analysis of composite material 902 during fabrication of a composite workpiece 200 includes the method 1700 of Fig. 17, the method 1800 of Fig. 18 and the method 1900 of Fig. 19. **In** this and certain other examples, the method 2000 continues from 1906 of Fig. 19 to 2002 where the design data 926 is optimized to form optimized design data 962 for the layup of subsequent layers 950 of the composite material 902 during the fabrication of the composite workpiece 200 to reduce the through thickness risk probability 972 of not achieving the fiber volume fill ratio requirements 952 for the composite workpiece 200 after the laying up 1706 of the subsequent layers 950. At 2004, the optimized design data 962 is processed for the subsequent layers 950 of the layup to determine as-planned fiber volume fill characteristics 928 of the subsequent layers 950. At 2006, a next layer 1606 of the composite material 902 is laid up over the current layers 1608 on the layup mandrel 942 based on the optimized design data 962.

In some examples of the method 2000, the optimizing 2002 of the design data 926 includes optimizing 2102 path planning for at least a portion of a remainder of the composite workpiece 200 to form an optimized path plan 964 for the layup of at least one subsequent layer 950 of the composite material 902 during the fabrication of the composite workpiece 200 to reduce the through thickness risk probability 972 of not achieving the fiber volume fill ratio requirements 952 for the composite workpiece 200 after the laying up 2006 of the at least one subsequent layer 950. In some examples of the method 2000, the optimizing 2002 of the design data 926 includes optimizing 2104 fiber volume fill ratio requirements 952 for at least a portion of a remainder of the composite workpiece 200 to form an optimized fiber volume fill ratio requirement 966 for the layup of at least one subsequent layer 950 of the composite material 902 during the fabrication of the composite workpiece 200 to reduce the through thickness risk probability 972 of not achieving the fiber volume fill ratio requirements 952 for the composite workpiece 200 after the laying up 2006 of the at least one subsequent layer 950. In some examples of the method 2000, the optimizing 2002 of the design data 926 includes optimizing 2106 numerical control programming for at least a portion of a remainder of the composite workpiece 200 to form an optimized numerical control program 968 for the layup of at least one subsequent layer 950 of the composite material 902 during the fabrication of the composite workpiece 200 to reduce the through thickness risk probability 972 of not achieving the fiber volume fill ratio requirements 952 for the composite workpiece 200 after the laying up 2006 of the at least one subsequent layer 950.

With reference again to Figs. 9, 17-19 and 22, in one or more examples, where the through thickness risk probability 972 for the current layers 1608 on the layup mandrel 942 does not exceed the risk threshold 958, the through thickness risk probability 972 is acceptable and a method 2200 (see Fig. 22) for layup and analysis of composite material 902 during fabrication of a composite workpiece 200 includes the method 1700 of Fig. 17, the method 1800 of Fig. 18 and the method 1900 of Fig. 19. In this and certain other examples, the method 2200 continues from 1906 of Fig. 19 to 2202 where the design data 926 for a next layer 1606 of the layup is processed to determine as-planned fiber volume fill characteristics 928 of the next layer 1606. At 2204, the next layer 1606 of the composite material 902 is laid up over the current layers 1608 on the layup mandrel 942 based on the design data 926.

Referring generally to Figs. 2, 9 and 23, by way of examples, the present disclosure is directed to a method 2300 for layup and analysis of composite materials 902 during fabrication of composite workpieces 200. Fig. 2 provides an example of a composite workpiece 200. Fig. 9 is a functional block diagram of another example of another automated system 900 for layup and analysis of composite material 902 during fabrication of a composite workpiece 200. Fig. 23 provides an example of the method 2300 for layup and analysis of composite material 902 during fabrication of a composite workpiece 200.

With reference again to Figs. 2, 9 and 23, in one or more examples, a method 2300 (see Fig. 23) for layup and analysis of composite material 902 during fabrication of a composite workpiece 200 includes laying up 2302 a layer 946 on a layup mandrel 942 based on design data 926. At 2304, as-built data 930 is detected using a quality detection device 944. At 2306, the as-built data 930 is processed to determine as-built fiber volume fill characteristics 932. At 2308, the as-built fiber volume fill characteristics 932 is compared to as-planned fiber volume fill characteristics 928 to determine projected fiber volume fill ratios 934 for the layer 946.

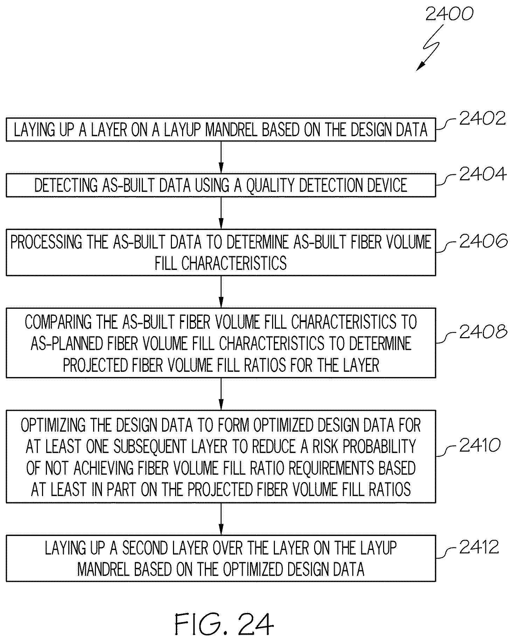

Referring generally to Figs. 2, 9, 16 and 24, by way of examples, the present disclosure is directed to a method 2400 for optimizing design data 926 during fabrication of a composite workpiece 200. Fig. 2 provides an example of a composite workpiece 200. Fig. 9 is a functional block diagram of another example of another automated system 900 for layup and analysis of composite material 902 during fabrication of a composite workpiece 200. Fig. 16 is a functional diagram showing an example of a grid structure 1600 for a composite workpiece 200 used in conjunction with layup and analysis of composite material 902 during fabrication of a composite workpiece 200. Fig. 24 provides an example of the method 2400 for optimizing design data 926 during fabrication of a composite workpiece 200.

With reference again to Figs. 2, 9, 16 and 24, in one or more examples, a method 2400 (see Fig. 24) for optimizing design data 926 during fabrication of a composite workpiece 200 includes laying up 2402 a layer 946 on a layup mandrel 942 based on the design data 926. At 2404, as-built data 930 is detected using a quality detection device 944. At 2406, the as-built data 930 is processed to determine as-built fiber volume fill characteristics 932. At 2408, the as-built fiber volume fill characteristics 932 is compared to as-planned fiber volume fill characteristics 928 to determine projected fiber volume fill ratios 934 for the layer 946. At 2410, the design data 926 is optimized to form optimized design data 962 for at least one subsequent layer 950 to reduce a risk probability 956 of not achieving fiber volume fill ratio requirements 952 based at least in part on the projected fiber volume fill ratios 934. At 2412, a second layer 1604 is laid up over the layer 946 on the layup mandrel 942 based on the optimized design data 962.

In some examples of the method 2400, the optimized design data 962 includes an optimized path plan 964, an optimized fiber volume fill ratio requirement 966, an optimized numerical control program 968 or any other suitable optimized design data in any suitable combination.

Examples of the methods 300, 400, 500, 600, 800, 1700, 1800, 1900, 2000, 2200, 2300 for layup and analysis of composite material 902 during fabrication of a composite workpiece 200, automated systems 100, 900 for layup and analysis of composite material 902 during fabrication of a composite workpiece 200 and methods 2400 for optimizing design data 926 during fabrication of a composite workpiece 200 may be related to or used in the context of aircraft manufacturing. Although an aircraft example is described, the examples and principles disclosed herein may be applied to other products in the aerospace industry and other industries, such as the automotive industry, the space industry, the construction industry and other design and manufacturing industries. Accordingly, in addition to aircraft, the examples and principles disclosed herein may apply to the use of various products in the manufacture of various types of vehicles and in the construction of various types of buildings.

The preceding detailed description refers to the accompanying drawings, which illustrate specific examples described by the present disclosure. Other examples having different structures and operations do not depart from the scope of the present disclosure. Like reference numerals may refer to the same feature, element, or component in the different drawings. Throughout the present disclosure, any one of a plurality of items may be referred to individually as the item and a plurality of items may be referred to collectively as the items and may be referred to with like reference numerals. Moreover, as used herein, a feature, element, component, or step preceded with the word "a" or "an" should be understood as not excluding a plurality of features, elements, components, or steps, unless such exclusion is explicitly recited.

Illustrative, non-exhaustive examples, which may be, but are not necessarily, claimed, of the subject matter according to the present disclosure are provided above. Reference herein to "example" means that one or more feature, structure, element, component, characteristic and/or operational step described in connection with the example is included in at least one aspect, embodiment and/or implementation of the subject matter according to the present disclosure. Thus, the phrases "an example," "another example," "one or more examples," and similar language throughout the present disclosure may, but do not necessarily, refer to the same example. Further, the subject matter characterizing any one example may, but does not necessarily, include the subject matter characterizing any other example. Moreover, the subject matter characterizing any one example may be, but is not necessarily, combined with the subject matter characterizing any other example.

As used herein, a system, apparatus, device, structure, article, element, component, or hardware "configured to" perform a specified function is indeed capable of performing the specified function without any alteration, rather than merely having potential to perform the specified function after further modification. In other words, the system, apparatus, device, structure, article, element, component, or hardware "configured to" perform a specified function is specifically selected, created, implemented, utilized, programmed and/or designed for the purpose of performing the specified function. As used herein, "configured to" denotes existing characteristics of a system, apparatus, structure, article, element, component, or hardware that enable the system, apparatus, structure, article, element, component, or hardware to perform the specified function without further modification. For purposes of this disclosure, a system, apparatus, device, structure, article, element, component, or hardware described as being "configured to" perform a particular function may additionally or alternatively be described as being "adapted to" and/or as being "operative to" perform that function.

Unless otherwise indicated, the terms "first," "second," "third," etc., are used herein merely as labels and are not intended to impose ordinal, positional, or hierarchical requirements on the items to which these terms refer. Moreover, reference to, e.g., a "second" item does not require or preclude the existence of, e.g., a "first" or lower-numbered item and/or, e.g., a "third" or higher-numbered item.

As used herein, the phrase "at least one of," when used with a list of items, means different combinations of one or more of the listed items may be used and only one of each item in the list may be needed. For example, "at least one of item A, item B and item C" may include, without limitation, item A or item A and item B. This example also may include item A, item B and item C or item B and item C. In other examples, "at least one of" may be, for example, without limitation, two of item A, one of item B and ten of item C; four of item B and seven of item C; and other suitable combinations. As used herein, the term "and/or" and the "/" symbol includes any and all combinations of one or more of the associated listed items.

As used herein, the terms "coupled," "coupling," and similar terms refer to two or more elements that are joined, linked, fastened, attached, connected, put in communication, or otherwise associated (e.g., mechanically, electrically, fluidly, optically, electromagnetically) with one another. In various examples, the elements may be associated directly or indirectly. As an example, element A may be directly associated with element B. As another example, element A may be indirectly associated with element B, for example, via another element C. It will be understood that not all associations among the various disclosed elements are necessarily represented. Accordingly, couplings other than those depicted in the figures may also exist.

As used herein, the term "approximately" refers to or represents a condition that is close to, but not exactly, the stated condition that still performs the desired function or achieves the desired result. As an example, the term "approximately" refers to a condition that is within an acceptable predetermined tolerance or accuracy, such as to a condition that is within 10% of the stated condition. However, the term "approximately" does not exclude a condition that is exactly the stated condition. As used herein, the term "substantially" refers to a condition that is essentially the stated condition that performs the desired function or achieves the desired result.

In Figs. 1, 2, 9, 10, 11A-C, 12A-B and 13-16, referred to above, may represent functional elements, features, or components thereof and do not necessarily imply any particular structure. Accordingly, modifications, additions and/or omissions may be made to the illustrated structure. Additionally, those skilled in the art will appreciate that not all elements, features and/or components described and illustrated in Figs. 1, 2, 9, 10, 11A-C, 12A-B and 13-16, referred to above, need be included in every example and not all elements, features and/or components described herein are necessarily depicted in each illustrative example. Accordingly, some of the elements, features and/or components described and illustrated in Figs. 1, 2, 9, 10, 11A-C, 12A-B and 13-16 may be combined in various ways without the need to include other features described and illustrated in Figs. 1, 2, 9, 10, 11A-C, 12A-B and 13-16, other drawing figures and/or the accompanying disclosure, even though such combination or combinations are not explicitly illustrated herein. Similarly, additional features not limited to the examples presented, may be combined with some or all the features shown and described herein. Unless otherwise explicitly stated, the schematic illustrations of the examples depicted in Figs. 1, 2, 9, 10, 11A-C, 12A-B and 13-16, referred to above, are not meant to imply structural limitations with respect to the illustrative example. Rather, although one illustrative structure is indicated, it is to be understood that the structure may be modified when appropriate. Accordingly, modifications, additions and/or omissions may be made to the illustrated structure. Furthermore, elements, features and/or components that serve a similar, or at least substantially similar, purpose are labeled with like numbers in each of Figs. 1, 2, 9, 10, 11A-C, 12A-B and 13-16 and such elements, features and/or components may not be discussed in detail herein with reference to each of Figs. 1, 2, 9, 10, 11A-C, 12A-B and 13-16. Similarly, all elements, features and/or components may not be labeled in each of Figs. 1, 2, 9, 10, 11A-C, 12A-B and 13-1613-16, but reference numerals associated therewith may be utilized herein for consistency.

In Figs. 3A-B, 4-8 and 17-24, referred to above, the blocks may represent operations, steps and/or portions thereof, and lines connecting the various blocks do not imply any particular order or dependency of the operations or portions thereof. It will be understood that not all dependencies among the various disclosed operations are necessarily represented. Figs. 3A-B, 4-8 and 17-24 and the accompanying disclosure describing the operations of the disclosed methods set forth herein should not be interpreted as necessarily determining a sequence in which the operations are to be performed. Rather, although one illustrative order is indicated, it is to be understood that the sequence of the operations may be modified when appropriate. Accordingly, modifications, additions and/or omissions may be made to the operations illustrated and certain operations may be performed in a different order or simultaneously. Additionally, those skilled in the art will appreciate that not all the operations described need to be performed.

Further, references throughout the present specification to features, advantages, or similar language used herein do not imply that all the features and advantages that may be realized with the examples disclosed herein should be, or are in, any single example. Rather, language referring to the features and advantages is understood to mean that a specific feature, advantage, or characteristic described in connection with an example is included in at least one example. Thus, discussion of features, advantages and similar language used throughout the present disclosure may, but does not necessarily, refer to the same example.

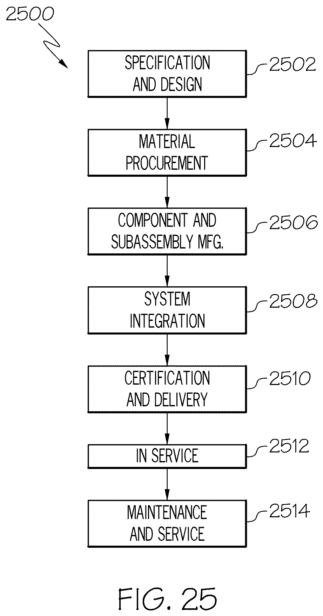

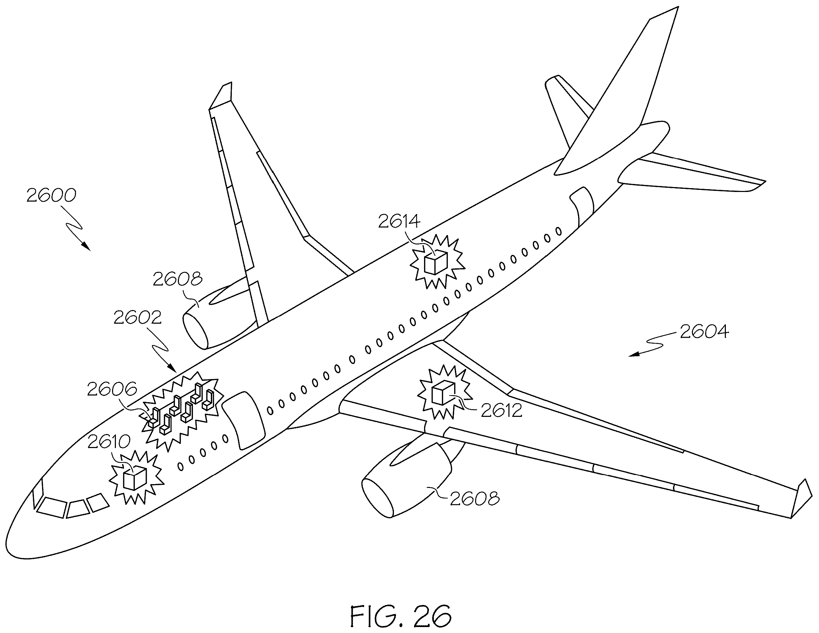

Examples of the subject matter disclosed herein may be described in the context of aircraft manufacturing and service method 2500 as shown in Fig. 25 and aircraft 2600 as shown in Fig. 26. **In** one or more examples, the disclosed automated systems 100, 900 for layup and analysis of composite material 902 during fabrication of a composite workpiece 200 and methods 2400 for optimizing design data 926 during fabrication of a composite workpiece 200 may be used in aircraft manufacturing. During pre-production, the service method 2500 may include specification and design (block 2502) of aircraft 2600 and material procurement (block 2504). During production, component and subassembly manufacturing (block 2506) and system integration (block 2508) of aircraft 2600 may take place. Thereafter, aircraft 2600 may go through certification and delivery (block 2510) to be placed in service (block 2512). While in service, aircraft 2600 may be scheduled for routine maintenance and service (block 2514). Routine maintenance and service may include modification, reconfiguration, refurbishment, etc. of one or more systems of aircraft 2600.

Each of the processes of the service method 2500 may be performed or carried out by a system integrator, a third party and/or an operator (e.g., a customer). For the purposes of this description, a system integrator may include, without limitation, any number of aircraft manufacturers and major-system subcontractors; a third party may include, without limitation, any number of vendors, subcontractors and suppliers; and an operator may be an airline, leasing company, military entity, service organization and so on.

As shown in Fig. 26, aircraft 2600 produced by the service method 2500 may include airframe 2602 with a plurality of high-level systems 2604 and interior 2606. Examples of high-level systems 2604 include one or more of propulsion system 2608, electrical system 2610, hydraulic system 2612 and environmental system 2614. Any number of other systems may be included. Although an aerospace example is shown, the principles disclosed herein may be applied to other industries, such as the automotive industry. Accordingly, in addition to aircraft 2600, the principles disclosed herein may apply to other vehicles, e.g., land vehicles, marine vehicles, space vehicles, etc.

The disclosed methods 300, 400, 500, 600, 800, 1700, 1800, 1900, 2000, 2200, 2300 for layup and analysis of composite material 902 during fabrication of a composite workpiece 200, automated systems 100, 900 for layup and analysis of composite material 902 during fabrication of a composite workpiece 200 and methods 2400 for optimizing design data 926 during fabrication of a composite workpiece 200 may be used in fabrication of aircraft components. For example, components or subassemblies corresponding to component and subassembly manufacturing (block 2506) may be fabricated or manufactured in a manner similar to components or subassemblies produced while aircraft 2600 is in service (block 2512). Also, one or more example of the system(s), method(s), or combination thereof may be utilized during production stages (block 2506 and block 2508), for example, by substantially expediting assembly of or reducing the cost of aircraft 2600. Similarly, one or more example of the system or method realizations, or a combination thereof, may be utilized, for example and without limitation, while aircraft 2600 is in service (block 2512) and/or during maintenance and service (block 2514).

The described features, advantages and characteristics of one example may be combined in any suitable manner in one or more other examples. One skilled in the relevant art will recognize that the examples described herein may be practiced without one or more of the specific features or advantages of a particular example. In other instances, additional features and advantages may be recognized in certain examples that may not be present in all examples. Furthermore, various examples of the methods 300, 400, 500, 600, 800, 1700, 1800, 1900, 2000, 2200, 2300 for layup and analysis of composite material 902 during fabrication of a composite workpiece 200, automated systems 100, 900 for layup and analysis of composite material 902 during fabrication of a composite workpiece 200 and methods 2400 for optimizing design data 926 during fabrication of a composite workpiece 200 have been shown and described, modifications may occur to those skilled in the art upon reading the specification. The present application includes such modifications and is limited only by the scope of the claims.

Further examples are set out in the following causes.

Clause 1. A method for layup and analysis of composite material during fabrication of a composite workpiece, comprising:
laying up a layer of the composite material for the composite workpiece based on design data for the layer;
detecting as-built data for the layer using a quality detection device;
processing the as-built data for the layer to determine as-built fiber volume fill characteristics of the layer; and
comparing the as-built fiber volume fill characteristics to as-planned fiber volume fill characteristics based on the design data for the layer to determine projected fiber volume fill ratios for the layer.

Clause 2. The method of clause 1 wherein the layer of the composite material is laid up on a layup mandrel in a fabrication cell, a second layer of the composite material is laid up over the layer and subsequent layers of the composite material are laid up over current layers.

Clause 3. The method of clause 1 or 2 wherein the detecting of the as-built data is performed while the layer is on a layup mandrel.

Clause 4. The method of any of clauses 1 to 3, further comprising:
laying up subsequent layers of the composite material for the composite workpiece based on the design data for the subsequent layers;
detecting as-built data for each subsequent layer after the laying up of the corresponding subsequent layer using a quality detection device;
processing the as-built data for the subsequent layers to determine subsequent as-built fiber volume fill characteristics for each of the subsequent layers; and
processing the as-built fiber volume fill characteristics for the layer and for each of the subsequent layers to determine through thickness as-built fiber volume fill characteristics.

Clause 5. The method of any of clauses 1 to 4 wherein the composite workpiece comprises multiple zones and the design data for the layup comprises zone locational information for at least a portion of the design data.

Clause 6. The method of any of clauses 1 to 5 wherein the layer comprises at least one of a ply of composite material, a plurality of tows of composite material, and a plurality of courses of tows of composite material.

Clause 7. The method of any of clauses 1 to 6 wherein the as-built data is divided into a grid structure for the composite workpiece that comprises a plurality of data cells for each layer of the composite workpiece and the as-built data comprises grid locational information.

Clause 8. The method of any of clauses 1 to 7, further comprising:
receiving design data for the layup of the composite material; and
processing the design data for the layer of the layup to determine as-planned fiber volume fill characteristics of the layer.

Clause 9. The method of any of clauses 1 to 8, further comprising:
receiving fiber volume fill ratio requirements for the layup; and
comparing the projected fiber volume fill ratios for the layer to the fiber volume fill ratio requirements to determine a risk probability of not achieving the fiber volume fill ratio requirements for the composite workpiece.

Clause 10. The method of clause 9 wherein the composite workpiece comprises multiple layers and the fiber volume fill ratio requirements for the layup comprises layer locational information for each layer of the composite workpiece individually and for the multiple layers collectively.

Clause 11. The method of clause 9 or 10, further comprising:
receiving a risk threshold for the risk probability to categorize the risk probability as acceptable or unacceptable; and
comparing the risk probability to the risk threshold for the risk probability.

Clause 12. The method of clause 11 wherein, where the risk probability exceeds the risk threshold, the risk probability is unacceptable, the method further comprising:
optimizing the design data to form optimized design data for the layup of at least one subsequent layer of the composite material during the fabrication of the composite workpiece to reduce the risk probability of not achieving the fiber volume fill ratio requirements for the composite workpiece;
processing the optimized design data for a second layer of the layup to determine as-planned fiber volume fill characteristics of the second layer; and
laying up a second layer of the composite material over the layer based on the optimized design data.

Clause 13. The method of clause 12, the optimizing of the design data comprising:
optimizing path planning for at least a portion of a remainder of the composite workpiece to form an optimized path plan for the layup of at least one subsequent layer of the composite material during the fabrication of the composite workpiece to reduce the risk probability of not achieving the fiber volume fill ratio requirements for the composite workpiece.

Clause 14. The method of clause 12 or 13, the optimizing of the design data comprising:
optimizing fiber volume fill ratio requirements for at least a portion of a remainder of the composite workpiece to form an optimized fiber volume fill ratio requirement for the layup of at least one subsequent layer of the composite material during the fabrication of the composite workpiece to reduce the risk probability of not achieving the fiber volume fill ratio requirements for the composite workpiece.

Clause 15. The method of any of clauses 12 to 14, the optimizing of the design data comprising:
optimizing numerical control programming for at least a portion of a remainder of the composite workpiece to form an optimized numerical control program for the layup of at least one subsequent layer of the composite material during the fabrication of the composite workpiece to reduce the risk probability of not achieving the fiber volume fill ratio requirements for the composite workpiece.

Clause 16. The method of any of clauses 11 to 15 wherein, where the risk probability does not exceed the risk threshold, the risk probability is acceptable, the method further comprising:
processing the design data for a second layer of the layup to determine as-planned fiber volume fill characteristics of the second layer; and
laying up the second layer of the composite material over the layer based on the design data.

Clause 17. An automated system for layup and analysis of composite material during fabrication of a composite workpiece, comprising:
at least one analytical computing device storing a design data analysis application program, an as-built data analysis application program and a projected fiber volume fill ratio application program;
a robotic manipulator in a fabrication cell and in operative communication with the at least one analytical computing device, the robotic manipulator comprising a composite material placement device;
a layup mandrel in the fabrication cell and configured for layup of the composite material; and
a quality detection device in the fabrication cell and in operative communication with the at least one analytical computing device.

Clause 18. The automated system of clause 17, the at least one analytical computing device, comprising:
a network interface in operative communication with a communication network;
at least one processor and associated memory, the at least one processor in operative communication with a design data storage device via the network interface and the communication network;
at least one application program storage device in operative communication with the at least one processor and storing the design data analysis application program, the as-built data analysis application program and the projected fiber volume fill ratio application program, the at least one application program storage device also storing one or more of a fiber volume fill risk application program and a design data optimization application program; and
at least one local data storage device configured to at least temporarily store design data, as-planned fiber volume fill characteristics, as-built data, as-built fiber volume fill characteristics and projected fiber volume fill ratios, the at least one local data storage device also configured to at least temporarily store one or more of fiber volume fill ratio requirements, risk probability, risk threshold, optimized path plan, optimized fiber volume fill ratio requirements and optimized numerical control program.

Clause 19. The automated system of clause 18 wherein the composite workpiece comprises multiple zones and the design data for the layup comprises zone locational information for at least a portion of the design data.

Clause 20. The automated system of any of clauses 17 to 19 wherein the composite material placement device comprises an automated fiber placement device.

Clause 21. The automated system of any of clauses 17 to 20 wherein the quality detection device comprises at least one of a profilometer and a vision system.

Clause 22. The automated system of any of clauses 17 to 21 wherein the robotic manipulator and the composite material placement device are configured to layup a layer of the composite material for the composite workpiece on the layup mandrel in the fabrication cell based on design data for the layer,
wherein, after the layup of the layer, the quality detection device is configured to detect as-built data for the layer on the layup mandrel,
wherein the at least one analytical computing device, in conjunction with running the as-built data analysis application program, is configured to process the as-built data for the layer to determine as-built fiber volume fill characteristics of the layer on the layup mandrel, and
wherein the at least one analytical computing device, in conjunction with running the projected fiber volume fill ratio application program, is configured to compare the as-built fiber volume fill characteristics to as-planned fiber volume fill characteristics based on the design data for the layer to determine projected fiber volume fill ratios for the layer.

Clause 23. The automated system of clauses22 wherein the as-built data is divided into a grid structure for the composite workpiece that comprises a plurality of data cells for each layer of the composite workpiece and the as-built data comprises grid locational information.

Clause 24. The automated system of clause 22 or 23 wherein the at least one analytical computing device is configured to receive design data for the layup of the composite material from a design data storage device via a network interface and a communication network, and
wherein the at least one analytical computing device, in conjunction with running the design data analysis application program, is configured to process the design data for the layer of the layup to determine as-planned fiber volume fill characteristics of the layer.

Clause 25. The automated system of any of clauses 22 to 24 wherein the at least one analytical computing device is configured to receive fiber volume fill ratio requirements for the layup from a design data storage device via a network interface and a communication network, and
wherein the at least one analytical computing device, in conjunction with running the fiber volume fill risk application program, is configured to compare the projected fiber volume fill ratios for the layer to the fiber volume fill ratio requirements to determine a risk probability of not achieving the fiber volume fill ratio requirements for the composite workpiece after the layup of the layer.

Clause 26. The automated system of clause 25 wherein the at least one analytical computing device is configured to receive a risk threshold for the risk probability to categorize the risk probability as acceptable or unacceptable from the design data storage device via a network interface and a communication network, and
wherein the at least one analytical computing device, in conjunction with running the fiber volume fill risk application program, is configured to compare the risk probability after the layup of the layer to the risk threshold for the risk probability.

Clause 27. The automated system of clause 26 wherein, where the risk probability exceeds the risk threshold, the risk probability is unacceptable,
wherein the at least one analytical computing device, in conjunction with running a design data optimization application program, is configured to optimize the design data to form optimized design data for the layup of at least one subsequent layer of the composite material during the fabrication of the composite workpiece to reduce the risk probability of not achieving the fiber volume fill ratio requirements for the composite workpiece after the layup of the at least one subsequent layer,
wherein the at least one analytical computing device, in conjunction with running the design data analysis application program, is configured to process the optimized design data for a second layer of the layup to determine as-planned fiber volume fill characteristics of the second layer, and
wherein the robotic manipulator and the composite material placement device are configured to layup a second layer of the composite material over the layer on the layup mandrel based on the optimized design data.

Clause 28. The automated system of clause 27 wherein the at least one analytical computing device, in conjunction with running the design data optimization application program, is configured to optimize path planning for at least a portion of a remainder of the composite workpiece to form an optimized path plan for the layup of at least one subsequent layer of the composite material during the fabrication of the composite workpiece to reduce the risk probability of not achieving the fiber volume fill ratio requirements for the composite workpiece after the layup of the at least one subsequent layer.

Clause 29. The automated system of clause 27 or 28 wherein the at least one analytical computing device, in conjunction with running the design data optimization application program, is configured to optimize fiber volume fill ratio requirements for at least a portion of a remainder of the composite workpiece to form an optimized fiber volume fill ratio requirement for the layup of at least one subsequent layer of the composite material during the fabrication of the composite workpiece to reduce the risk probability of not achieving the fiber volume fill ratio requirements for the composite workpiece after the layup of the at least one subsequent layer.

Clause 30. The automated system of any of clauses 27 to 29 wherein the at least one analytical computing device, in conjunction with running the design data optimization application program, is configured to optimize numerical control programming for at least a portion of a remainder of the composite workpiece to form an optimized numerical control program for the layup of at least one subsequent layer of the composite material during the fabrication of the composite workpiece to reduce the risk probability of not achieving the fiber volume fill ratio requirements for the composite workpiece after the layup of the at least one subsequent layer.

Clause 31. The automated system of any of clauses 26 to 30 wherein, where the risk probability does not exceed the risk threshold, the risk probability is acceptable,
wherein the at least one analytical computing device, in conjunction with running the design data analysis application program, is configured to process the design data for a second layer of the layup to determine as-planned fiber volume fill characteristics of the second layer, and
wherein the robotic manipulator and the composite material placement device are configured to layup the second layer of the composite material over the layer on the layup mandrel based on the design data.

Clause 32. A method for layup and analysis of composite material during fabrication of a composite workpiece, comprising:
laying up a layer of multiple layers of the composite material for the composite workpiece based on design data for the layer;
detecting as-built data for the layer using a quality detection device;
processing the as-built data for the layer to determine as-built fiber volume fill characteristics of the layer;
comparing the as-built fiber volume fill characteristics for the layer to as-planned fiber volume fill characteristics based on the design data for the layer to determine projected fiber volume fill ratios for the layer;
repeating the laying up, the detecting, the processing, and the comparing for each subsequent layer of the multiple layers; and
processing the projected fiber volume fill ratios for the multiple layers to determine at least one through thickness projected fiber volume fill ratio.

Clause 33. The method of clause 32 wherein the composite workpiece comprises multiple zones and the design data for the layup comprises zone locational information for at least a portion of the design data.

Clause 34. The method of clause 32 or 33 wherein the as-built data is divided into a grid structure for the composite workpiece that comprises a plurality of data cells for each layer of the multiple layers and the as-built data comprises grid locational information.

Clause 35. The method of any of clauses 32 to 34, further comprising:
receiving design data for the layup of multiple layers of the composite material in the composite workpiece; and
processing the design data for the multiple layers to determine as-planned fiber volume fill characteristics for each layer individually and for the multiple layers collectively.

Clause 36. The method of any of clauses 32 to 35, further comprising:
receiving fiber volume fill ratio requirements for each layer individually and for the multiple layers collectively;
comparing the projected fiber volume fill ratios for each layer individually to the fiber volume fill ratio requirements for the corresponding layer to determine a risk probability of not achieving the fiber volume fill ratio requirements for the composite workpiece after the laying up of each layer; and
after the laying up of each subsequent layer, processing the risk probability for each layer to determine a through thickness risk probability for current layers on a layup mandrel.

Clause 37. The method of clause 36, further comprising:
receiving a risk threshold for the risk probability at each layer individually and for the multiple layers collectively to categorize the risk probability as acceptable or unacceptable;
comparing the risk probability after the laying up of each layer to the risk threshold for the risk probability at the corresponding layer; and
after the laying up of each subsequent layer, processing the risk probability for each layer to determine a through thickness risk probability for the current layers and comparing the through thickness risk probability to the corresponding risk threshold for the current layers on the layup mandrel.

Clause 38. The method of clause 37 wherein, where the through thickness risk probability for the current layers on the layup mandrel exceeds the risk threshold, the through thickness risk probability is unacceptable, the method further comprising:
optimizing the design data to form optimized design data for the layup of subsequent layers of the composite material during the fabrication of the composite workpiece to reduce the through thickness risk probability of not achieving the fiber volume fill ratio requirements for the composite workpiece after the laying up of the subsequent layers;
processing the optimized design data for the subsequent layers of the layup to determine as-planned fiber volume fill characteristics of the subsequent layers; and
laying up a next layer of the composite material over the current layers on the layup mandrel based on the optimized design data.

Clause 39. The method of clause 38, the optimizing of the design data comprising:
optimizing path planning for at least a portion of a remainder of the composite workpiece to form an optimized path plan for the layup of at least one subsequent layer of the composite material during the fabrication of the composite workpiece to reduce the through thickness risk probability of not achieving the fiber volume fill ratio requirements for the composite workpiece after the laying up of the at least one subsequent layer.

Clause 40. The method of clause 38 or 39, the optimizing of the design data comprising:
optimizing fiber volume fill ratio requirements for at least a portion of a remainder of the composite workpiece to form an optimized fiber volume fill ratio requirement for the layup of at least one subsequent layer of the composite material during the fabrication of the composite workpiece to reduce the through thickness risk probability of not achieving the fiber volume fill ratio requirements for the composite workpiece after the laying up of the at least one subsequent layer.

Clause 41. The method of any of clauses 38 to 40, the optimizing of the design data comprising:
optimizing numerical control programming for at least a portion of a remainder of the composite workpiece to form an optimized numerical control program for the layup of at least one subsequent layer of the composite material during the fabrication of the composite workpiece to reduce the through thickness risk probability of not achieving the fiber volume fill ratio requirements for the composite workpiece after the laying up of the at least one subsequent layer.

Clause 42. The method of any of clauses 37 to 41 wherein, where the through thickness risk probability for the current layers on the layup mandrel does not exceed the risk threshold, the through thickness risk probability is acceptable, the method further comprising:
processing the design data for a next layer of the layup to determine as-planned fiber volume fill characteristics of the next layer; and
laying up the next layer of the composite material over the current layers on the layup mandrel based on the design data.

Clause 43. A method for layup and analysis of composite material during fabrication of a composite workpiece, comprising:
laying up a layer on a layup mandrel based on design data;
detecting as-built data using a quality detection device;
processing the as-built data to determine as-built fiber volume fill characteristics; and
comparing the as-built fiber volume fill characteristics to as-planned fiber volume fill characteristics to determine projected fiber volume fill ratios for the layer.

Clause 44. An automated system for layup and analysis of composite material during fabrication of a composite workpiece, comprising:
an analytical computing device storing a design data analysis application program, an as-built data analysis application program and a projected fiber volume fill ratio application program;
a composite material placement device in operative communication with the analytical computing device;
a layup mandrel configured for layup of the composite material; and
a quality detection device in operative communication with the analytical computing device.

Clause 45. A method for optimizing design data during fabrication of a composite workpiece, comprising:
laying up a layer on a layup mandrel based on the design data;
detecting as-built data using a quality detection device;
processing the as-built data to determine as-built fiber volume fill characteristics;
comparing the as-built fiber volume fill characteristics to as-planned fiber volume fill characteristics to determine projected fiber volume fill ratios for the layer;
optimizing the design data to form optimized design data for at least one subsequent layer to reduce a risk probability of not achieving fiber volume fill ratio requirements based at least in part on the projected fiber volume fill ratios; and
laying up a second layer over the layer on the layup mandrel based on the optimized design data.

## Claims

1. An automated system (900) for layup and analysis of composite material (902) during fabrication of a composite workpiece (200), comprising:
at least one analytical computing device (904) storing a design data analysis application program (918), an as-built data analysis application program (920) and a projected fiber volume fill ratio application program (922);
a robotic manipulator (936) in a fabrication cell (938) and in operative communication with the at least one analytical computing device (904), the robotic manipulator (936) comprising a composite material placement device (940);
a layup mandrel (942) in the fabrication cell (938) and configured for layup of the composite material (902); and
a quality detection device (944) in the fabrication cell (938) and in operative communication with the at least one analytical computing device (904).

2. The automated system of claim 1, the at least one analytical computing device (904), comprising:
a network interface (906) in operative communication with a communication network (908);
at least one processor (910) and associated memory (912), the at least one processor (910) in operative communication with a design data storage device (914) via the network interface (906) and the communication network (908);
at least one application program storage device (916) in operative communication with the at least one processor (910) and storing the design data analysis application program (918), the as-built data analysis application program (920) and the projected fiber volume fill ratio application program (922), the at least one application program storage device (916) also storing one or more of a fiber volume fill risk application program (954) and a design data optimization application program (960); and
at least one local data storage device (924) configured to at least temporarily store design data (926), as-planned fiber volume fill characteristics (928), as-built data (930), as-built fiber volume fill characteristics (932) and projected fiber volume fill ratios (934), the at least one local data storage device (924) also configured to at least temporarily store one or more of fiber volume fill ratio requirements, risk probability, risk threshold, optimized path plan, optimized fiber volume fill ratio requirements and optimized numerical control program.

3. The automated system of claim 2 wherein the composite workpiece (200) comprises multiple zones (202) and the design data (926) for the layup comprises zone locational information (1012) for at least a portion of the design data (926).

4. The automated system of any preceding claim wherein the composite material placement device (940) comprises an automated fiber placement device (1302).

5. The automated system of any preceding claim wherein the quality detection device (944) comprises at least one of a profilometer (1502) and a vision system (1504).

6. The automated system any preceding claim wherein the robotic manipulator (936) and the composite material placement device (940) are configured to layup a layer (946) of the composite material (902) for the composite workpiece (200) on the layup mandrel (942) in the fabrication cell (938) based on design data (926) for the layer (946),
wherein, after the layup of the layer (946), the quality detection device (944) is configured to detect as-built data (930) for the layer (946) on the layup mandrel (942),
wherein the at least one analytical computing device (904), in conjunction with running the as-built data analysis application program (920), is configured to process the as-built data (930) for the layer (946) to determine as-built fiber volume fill characteristics (932) of the layer (946) on the layup mandrel (942), and
wherein the at least one analytical computing device (904), in conjunction with running the projected fiber volume fill ratio application program (922), is configured to compare the as-built fiber volume fill characteristics (932) to as-planned fiber volume fill characteristics (928) based on the design data (926) for the layer (946) to determine projected fiber volume fill ratios (934) for the layer (946).

7. The automated system of claim 6 wherein the as-built data (930) is divided into a grid structure (1600) for the composite workpiece (200) that comprises a plurality of data cells (1602) for each layer (946, 950) of the composite workpiece (200) and the as-built data (930) comprises grid locational information (1404).

8. The automated system of claim 6 or 7 wherein the at least one analytical computing device (904) is configured to receive design data (926) for the layup of the composite material (902) from a design data storage device (914) via a network interface (906) and a communication network (908), and
wherein the at least one analytical computing device (904), in conjunction with running the design data analysis application program (918), is configured to process the design data (926) for the layer (946) of the layup to determine as-planned fiber volume fill characteristics (928) of the layer (946).

9. The automated system of any of claims 6 to 8 wherein the at least one analytical computing device (904) is configured to receive fiber volume fill ratio requirements (952) for the layup from a design data storage device (914) via a network interface (906) and a communication network (908), and
wherein the at least one analytical computing device (904), in conjunction with running the fiber volume fill risk application program (954), is configured to compare the projected fiber volume fill ratios (934) for the layer (946) to the fiber volume fill ratio requirements (952) to determine a risk probability (956) of not achieving the fiber volume fill ratio requirements (952) for the composite workpiece (200) after the layup of the layer (946).

10. The automated system of claim 9 wherein the at least one analytical computing device (904) is configured to receive a risk threshold (958) for the risk probability (956) to categorize the risk probability (956) as acceptable or unacceptable from the design data storage device (914) via a network interface (906) and a communication network (908), and
wherein the at least one analytical computing device (904), in conjunction with running the fiber volume fill risk application program (954), is configured to compare the risk probability (956) after the layup of the layer (946) to the risk threshold (958) for the risk probability (956).

11. The automated system of claim 10 wherein, where the risk probability (956) exceeds the risk threshold (958), the risk probability (956) is unacceptable,
wherein the at least one analytical computing device (904), in conjunction with running a design data optimization application program (960), is configured to optimize the design data (926) to form optimized design data (962) for the layup of at least one subsequent layer (950) of the composite material (902) during the fabrication of the composite workpiece (200) to reduce the risk probability (956) of not achieving the fiber volume fill ratio requirements (952) for the composite workpiece (200) after the layup of the at least one subsequent layer (950),
wherein the at least one analytical computing device (904), in conjunction with running the design data analysis application program (918), is configured to process the optimized design data (962) for a second layer (1604) of the layup to determine as-planned fiber volume fill characteristics (928) of the second layer (1604), and
wherein the robotic manipulator (936) and the composite material placement device (940) are configured to layup a second layer (1604) of the composite material (902) over the layer (946) on the layup mandrel (942) based on the optimized design data (962).

12. The automated system of claim 11 wherein the at least one analytical computing device (904), in conjunction with running the design data optimization application program (960), is configured:
to optimize path planning for at least a portion of a remainder of the composite workpiece (200) to form an optimized path plan (964) for the layup of at least one subsequent layer (950) of the composite material (902) during the fabrication of the composite workpiece (200) to reduce the risk probability (956) of not achieving the fiber volume fill ratio requirements (952) for the composite workpiece (200) after the layup of the at least one subsequent layer (950); and/or
to optimize fiber volume fill ratio requirements (952) for at least a portion of a remainder of the composite workpiece (200) to form an optimized fiber volume fill ratio requirement (966) for the layup of at least one subsequent layer (950) of the composite material (902) during the fabrication of the composite workpiece (200) to reduce the risk probability (956) of not achieving the fiber volume fill ratio requirements (952) for the composite workpiece (200) after the layup of the at least one subsequent layer (950).

13. The automated system of any of claims 11 or 12 wherein the at least one analytical computing device (904), in conjunction with running the design data optimization application program (960), is configured to optimize numerical control programming for at least a portion of a remainder of the composite workpiece (200) to form an optimized numerical control program (968) for the layup of at least one subsequent layer (950) of the composite material (902) during the fabrication of the composite workpiece (200) to reduce the risk probability (956) of not achieving the fiber volume fill ratio requirements (952) for the composite workpiece (200) after the layup of the at least one subsequent layer (950).

14. The automated system of any of claims 10 to 13 wherein, where the risk probability (956) does not exceed the risk threshold (958), the risk probability (956) is acceptable,
wherein the at least one analytical computing device (904), in conjunction with running the design data analysis application program (918), is configured to process the design data (926) for a second layer (1604) of the layup to determine as-planned fiber volume fill characteristics (928) of the second layer (1604), and
wherein the robotic manipulator (936) and the composite material placement device (940) are configured to layup the second layer (1604) of the composite material (902) over the layer (946) on the layup mandrel (942) based on the design data (926).

15. A method (300) for layup and analysis of composite material (902) during fabrication of a composite workpiece (200), comprising:
laying up (306) a layer (946) of the composite material (902) for the composite workpiece (200) based on design data (926) for the layer (946);
detecting (308) as-built data (930) for the layer (946) using a quality detection device (944);
processing (310) the as-built data (930) for the layer (946) to determine as-built fiber volume fill characteristics (932) of the layer (946); and
comparing (312) the as-built fiber volume fill characteristics (932) to as-planned fiber volume fill characteristics (928) based on the design data (926) for the layer (946) to determine projected fiber volume fill ratios (934) for the layer (946).
